(19) **Europäisches Patentamt**
**European Patent Office**
**Office européen des brevets**

(11) **EP 3 946 147 B1**

(12) **EUROPEAN PATENT SPECIFICATION**

(45) Date of publication and mention
of the grant of the patent:
**17.12.2025 Bulletin 2025/51**

(21) Application number: **20709770.0**

(22) Date of filing: **17.02.2020**

(51) International Patent Classification (IPC):
*A61F 2/02* *(2006.01)* *B33Y 10/00* *(2015.01)*
*A61F 2/12* *(2006.01)* *B29C 64/00* *(2017.01)*
*B33Y 80/00* *(2015.01)* *A61F 2/08* *(2006.01)*
*B29C 64/106* *(2017.01)*

(52) Cooperative Patent Classification (CPC):
**A61F 2/022; A61F 2/08; A61F 2/12; B29C 64/106;**
**B33Y 10/00; B33Y 80/00;** A61F 2240/001

(86) International application number:
**PCT/SE2020/050176**

(87) International publication number:
**WO 2020/204777 (08.10.2020 Gazette 2020/41)**

(54) **SCAFFOLDS FOR USE IN TISSUE ENGINEERING AND METHOD FOR PREPARING SCAFFOLDS**

GERÜSTE ZUR VERWENDUNG IN DER GEWEBEZÜCHTUNG UND VERFAHREN ZUR
HERSTELLUNG VON GERÜSTEN

ÉCHAFAUDAGES DESTINÉS À ÊTRE UTILISÉS EN INGÉNIERIE TISSULAIRE ET PROCÉDÉ DE
PRÉPARATION D'ÉCHAFAUDAGES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **29.03.2019 SE 1950395**

(43) Date of publication of application:
**09.02.2022 Bulletin 2022/06**

(73) Proprietor: **Akira Science AB**
**114 26 Stockholm (SE)**

(72) Inventors:
• **LIU, Hailong**
**114 17 Stockholm (SE)**
• **FUOCO, Tiziana**
**117 34 Stockholm (SE)**
• **GASSER, Christian**
**182 74 Stocksund (SE)**
• **WISTRAND, Anna**
**162 71 Vällingby (SE)**

(74) Representative: **Brann AB**
**P.O. Box 3690**
**Sveavägen 63**
**103 59 Stockholm (SE)**

(56) References cited:
WO-A1-2014/095872 WO-A2-2006/091921
CA-A1- 3 042 433 CN-A- 109 385 393
US-A- 5 518 680 US-A1- 2016 374 431
US-A1- 2021 186 678

• **VIACHESLAV SLESARENKO ET AL: "Distinct**
**failure modes in bio-inspired 3D-printed**
**staggered composites under non-aligned**
**loadings", SMART MATERIALS AND**
**STRUCTURES, IOP PUBLISHING LTD., BRISTOL,**
**GB, vol. 26, no. 3, 17 February 2017 (2017-02-17),**
**pages 35053, XP020313964, ISSN: 0964-1726,**
**[retrieved on 20170217], DOI: 10.1088/1361-665X/**
**AA59EB**

**(Cont. next page)**

- **BAS ONUR ET AL: "The quest for mechanically and biologically functional soft biomaterialsviasoft network composites", ADVANCED DRUG DELIVERY REVIEWS, ELSEVIER, AMSTERDAM, NL, vol. 132, 24 July 2018 (2018-07-24), pages 214 - 234, XP085525248, ISSN: 0169-409X, DOI: 10.1016/J.ADDR.2018.07.015**

- **SEEMA AGARWAL ET AL: "Use of electrospinning technique for biomedical applications", POLYMER, vol. 49, no. 26, 1 December 2008 (2008-12-01), GB, pages 5603 - 5621, XP055652764, ISSN: 0032-3861, DOI: 10.1016/j.polymer.2008.09.014**

**Description**

FIELD OF THE DISCLOSURE

The present disclosure relates to the field of medical technology, and in particular is directed to scaffolds for use in tissue engineering, a method for preparing scaffolds and uses of scaffolds for various medical applications *in vivo* and *in vitro.*

BACKGROUND OF THE DISCLOSURE

In tissue engineering, scaffolds have been proven their values for the repair of injury and regeneration of damaged tissues. A three-dimensional (3D) porous scaffold serves as a supporting framework for cell attachment, cell growth, and tissue regeneration. In order to facilitate soft tissue reconstruction into the constructs, scaffolds should closely mimic the biological environment surrounding the target tissue. To achieve this, scaffolds need to possess proper properties (mechanical, chemical and biological) so that they can deliver biological cues that cells can sense and respond to. Studies reveal that structure is a key aspect that influences the performance of a scaffold. Among the key structural parameters, porosity is one of the most important. For example, Perez et al [Perez et al, Materials Science and Engineering C, 61: 922-939, 2016] have reported the importance of both macro- and micro-porosity and the role of the overall architecture and morphology of the pores on the mechanical properties of printed scaffolds. The mechanical properties are also function of the design of the scaffolds [Slesarenko et al, Smart materials and structures, 26(3): 35053, 2017]. 3D devices with customized properties to the need of application can be manufactured by using 3D printing as method and by varying patterns, materials or combination of them [US2016374431 A1]. For example, the mechanical properties can be varied by printing grid-like pattern staggered or offset from one other [WO2014095872 A1]. A device according to the preamble of claim 1 is known from the document US2021186678 A1.

The main problem when 3D-printing for medical applications today is that the scaffold becomes very stiff and the polymer degrades while printing if degradable polymers like poly(L-lactide), PLLA, poly(D-lactide), PDLA, poly(D,L-lactide), PDLLA, poly(meso-lactide), PmLA, poly(glycolide), PGA, poly(p-dioxanone) (PDX) and copolymers prepared by combinations of the relative monomers are printed. Poly($\varepsilon$-caprolactone), PCL, does not degrade since the melting temperature is low and it has higher thermal stability compared to the other aliphatic polyesters, but the degradation time *in vivo* of high molecular weight PCL is too long to be adequate for medical applications.

Thus, there is a need in the art for improved scaffolds, which are not degraded during manufacture but which are nevertheless degradable when implanted *in vivo* in an individual, and which scaffolds have a decreased stiffness and an increased permeability to achieve improved cell attachment, cell growth, and tissue regeneration.

SUMMARY OF THE DISCLOSURE

The above objective to provide improved scaffolds for medical applications is achieved by the present disclosure, which provides soft, degradable 3D-printable scaffolds by combining a gradient design and a staggered design of the scaffold.

More particularly, the present disclosure is directed to a scaffold for use in tissue engineering, comprising:

n layers, where n is an integer $\geq 4$;
wherein each layer extends in an x-y-plane;
wherein each layer has a height (H) in a z-direction perpendicular to the x-y-plane;
wherein each layer comprises m volume-building components comprising scaffold material and being aligned in r rows in the x-y-plane, where m is an integer $\geq 2$, where r is an integer $\geq 2$;
wherein each volume-building component in any row of a layer is positioned at a distance (Dx) in the x-direction from any adjacent volume-building component in any adjacent row of the same layer; wherein the m volume-building components of any group of layers are distributed at an angle ($\alpha$) in the x-y-plane relative to the m volume-building components of any adjacent group of layers; wherein the volume-building components are configured to allow biological cells to attach thereto;
**characterised in that**
the scaffold comprises a gradient distribution of volume-building components in the z-direction, wherein the distance (Dx) between any two adjacent groups of volume-building components of any group of layers is different from the distance (Dx) between any two adjacent groups of volume-building components of any adjacent group of layers; and that
the scaffold comprises a staggered distribution of volume-building components of a layer in the x-y-plane, wherein each volume-building component in any row of a layer is positioned at a distance (Dysr) in the y direction from any adjacent volume-building component in the same row, and wherein each group of volume-building components in any group of rows of a layer is positioned at a distance (Dyar) in the y-direction from any adjacent group of volume-building components in any adjacent group of rows of the same layer;

wherein the height (H), the distance (Dx), the distance (Dysr), the distance (Dyar) and the angle ($\alpha$) together define (or determine) a size and shape of pores in the scaffold, which pores are configured to allow biological cells to pass through the scaffold. Dysr is not always present. It presents when there are at least two volume-building components in a row at a layer.

Further disclosed is a medical device for use in tissue engineering, comprising a scaffold as disclosed herein.

The present disclosure also provides a method for preparing a scaffold as described above by additive manufacturing, e.g. 3D-printing, in an apparatus configured for additive manufacturing.

The present disclosure is also directed to a method for *in vivo* tissue engineering, such as for breast reconstruction after mastectomy, preferably for supporting a breast prosthesis, or for reconstruction of tendon/ligament or muscle junction in an individual, comprising implanting a scaffold or a medical device presently disclosed into the individual.

Further provided herein is a use of the presently disclosed scaffold in an *in vitro* cell culture system, in an *in vitro* method for culturing of cells and/or in an *in vitro* method for regenerating tissue.

Also provided is a scaffold and a medical device for use in a method for *in vivo* tissue engineering.

Further disclosed is a novel degradable copolymer of ε-caprolactone and p-dioxanone, which can be printed without degradation and which is particularly suitable for use as scaffold material in the scaffold and method according to the present disclosure.

Preferred aspects of the present disclosure are further described below in the detailed description and in the dependent claims.

BRIEF DESCRIPTION OF THE DRAWINGS

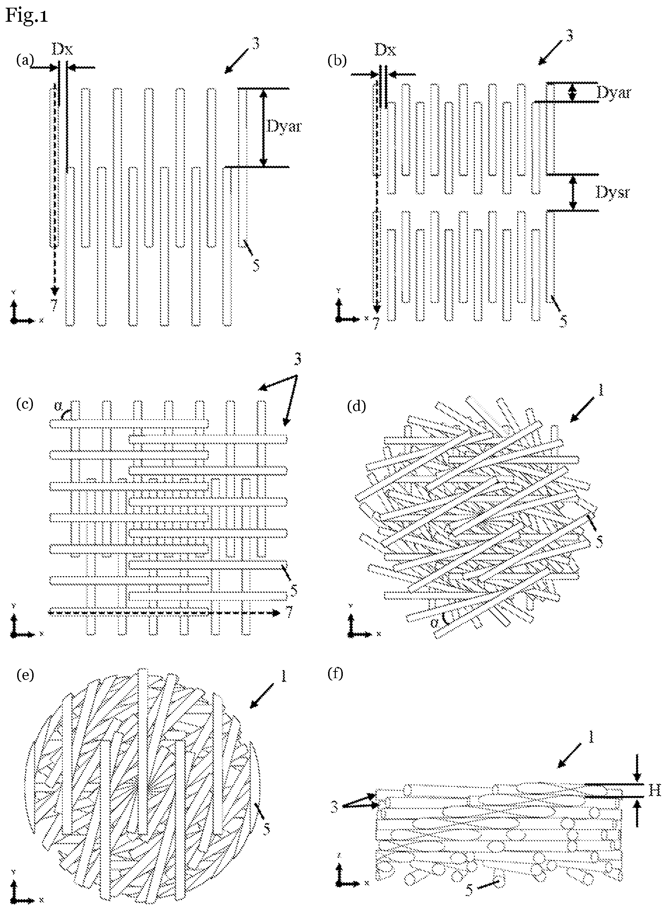

Fig. 1(a)-(c) schematically show different views of non-limiting embodiments of parts of a scaffold according to the present disclosure. Fig. 1(a) is a top view of one layer of a scaffold, the layer comprising a plurality of rows, each row comprising one volume-building component. Fig. 1(b) is a top view of one layer of a scaffold, the layer comprising a plurality of rows, each row comprising two volume-building components. Fig. 1(c) is a top view of two layers of a scaffold, each layer comprising a plurality of rows, each row comprising one volume-building component. Fig. 1(d)-(f) shows different views of non-limiting embodiments of a scaffold according to the present disclosure. Fig. 1(d) is a top view of a scaffold comprising a plurality of layers, each layer comprising a plurality of rows, each row comprising one volume-building component. Fig. 1(e) is a top view of a cylindrical scaffold comprising a plurality of layers, each layer comprising a plurality of rows, each row comprising one volume-building component. Fig. 1(f) is a side view of the cylindrical scaffold shown in fig. 1(e).

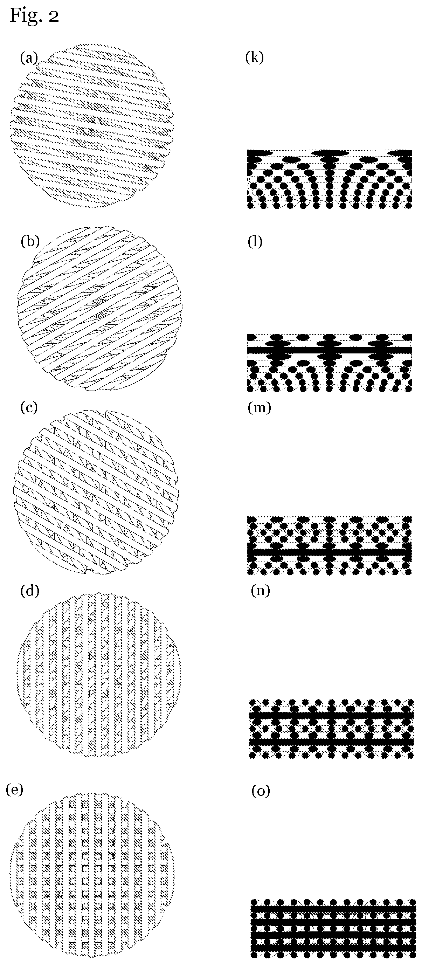

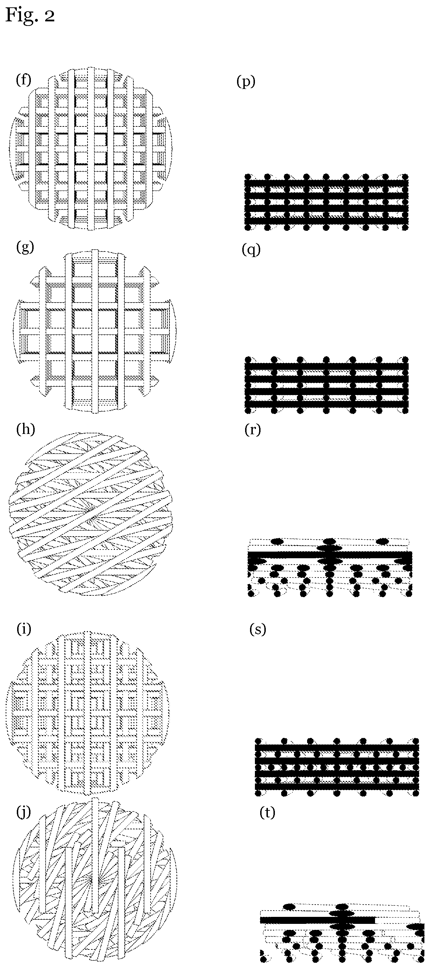

Fig. 2 (a)-(t) schematically shows different views and embodiments of a scaffold according to the present disclosure, as described in detail in Example 1 below.

Fig. 3 is a flow chart of a method for preparing a scaffold according to the present disclosure.

Fig. 4 summarizes the effective compressive/tensile moduli for each scaffold predicted from computational analysis.

Fig. 5 depicts the relation between effective compressive/tensile moduli and the porosity for all scaffolds.

Fig. 6 shows the comparison between experimental and computational compressive moduli for scaffolds (error bars show standard deviation, n=7).

Fig. 7 depicts the relation between computational normalized permeability and porosity for all scaffolds.

Fig. 8 shows micro-CT images of 3d printed scaffolds.

Fig. 9 shows the comparison of the porosity between the design and the micro-CT analysis for scaffolds (error bars denote standard deviation, n=3).

Fig. 10 illustrates the pore size distribution for printed scaffolds obtained from micro-CT analysis.

DETAILED DESCRIPTION OF THE DISCLOSURE

The present disclosure solves or at least mitigates the problems associated with existing scaffolds for use in medical applications by providing, as illustrated in Fig. 1, a scaffold 1 configured for use in tissue engineering, comprising:

> n layers 3, where n is an integer $\geq 4$;
> wherein each layer 3 extends in an x-y-plane;
> wherein each layer 3 has a height (H) in a z-direction perpendicular to the x-y-plane;
> wherein each layer 3 comprises m volume-building components 5 comprising scaffold material and being aligned in r rows 7 in the x-y-plane, where m is an integer $\geq 2$, where r is an integer $\geq 2$; wherein each volume-building component 5 in any row 7 of a layer 3 is positioned at a distance (Dx) in the x-direction from any adjacent volume-building component 5 in any adjacent row 7 of the same layer 3;
> wherein the m volume-building components 5 of any group of layers 3 are distributed at an angle ($\alpha$) in the x-y-plane relative to the m volume-building components 5 of any adjacent group of layers 3; wherein the volume-building components 5 are configured to allow biological cells to attach thereto;
> **characterised in that**
> the scaffold 1 comprises a gradient distribution of volume-building components 5 in the z-direction, wherein the distance (Dx) between any two adjacent groups of volume-building components 5 of any group of layers 3 is different from the distance (Dx) between any two adjacent groups of volume-building components 5 of any adjacent group of layers 3;
> and that
> the scaffold 1 comprises a staggered distribution of volume-building components 5 of a layer 3 in the x-y-plane, wherein each volume-building component 5 in any row 7 of a layer 3 is positioned at a distance (Dysr) in the y direction from any adjacent volume-building component 5 in the same row 7, and wherein each group of volume-building components 5 in any group of rows 7 of a layer 3 is positioned at a distance (Dyar) in the y-direction from any adjacent group of volume-building components 5 in any adjacent group of rows 7 of the same layer 3;
> wherein the height (H), the distance (Dx), the distance (Dysr), the distance (Dyar) and the angle ($\alpha$) together define (or determine) a size and shape of pores in the scaffold 1, which pores are configured to allow biological cells to pass through the scaffold.

The shape and the size of pores can be precisely tailored by combining different values of Dx, Dysr, Dyar and $\alpha$. Thereafter, the pores in the scaffold can be efficiently fabricated using additive manufacturing to possess desired characterization, such as shape and size distribution. This technology shows a huge advantage to other conventional fabrication methods (salt leaching, phase separation and gas foaming) in terms of high controllability and good reproducibility. The reason is that additive manufacturing uses data computer-aided-design (CAD) software or 3D object scanners to direct hardware to deposit material, layer upon layer, in precise geometric shape. For instance, the scaffold can be built from intercrossing struts deposited in the horizontal plane and form pores surrounded by struts with the aid of fused deposition modeling (FDM) technology. The complexity of the pore shape and size can be handled by FDM fabrication of a multi-layer design.

A significant advantage of the presently disclosed scaffold is that, as shown herein, the combination design of a gradient structure and a staggered structure can significantly improve the mechanical properties of a scaffold. Moreover, the reduction of the mechanical resistance of a scaffold to a given deformation can take place in both the z-direction (compression) and the x-y-plane (tension). Furthermore, the disclosed scaffold can possess a high porosity and a high permeability, which allows an efficient cell migration, as well as the transport of nutrients and waste.

Fig. 1(a)-(c) schematically shows different views of non-limiting embodiments of parts of a scaffold according to the present disclosure. Fig. 1(a) is a top view of one layer 3 of a scaffold, the layer 3 comprising a plurality of rows 7, each row 7 comprising one volume-building component 5. Dx is the distance in the x-direction between two adjacent volume-building components 5, located in adjacent rows 7. Dyar is the distance in the y-direction between two adjacent volume-building components 5 in adjacent rows 7 of the same layer 3. Fig. 1(b) is a top view of one layer 3 of a scaffold, the layer 3 comprising a plurality of rows 7, each row 7 comprising two volume-building components 5. Dysr is the distance in the y-direction between two volume-building components 5 in the same row 7. Dysr is not always present. It presents when there are at least two volume-building components 5 in a row at a layer. Fig. 1(c) is a top view of two layers 3 of a scaffold, each layer 3 comprising a plurality of rows 7, each row 7 comprising one volume-building component 5. Further, $\alpha$ is the angle in the x-y-plane between the orientation of volume-building components 5 of two adjacent layers 3. Fig. 1(d)-(f) shows different views of non-limiting embodiments of a scaffold according to the present disclosure. Fig. 1(d) is a top view of a scaffold 1 comprising a plurality of layers, each layer comprising a plurality of rows, each row comprising one volume-building component. Fig. 1(e) is a top view of a cylindrical scaffold 1 comprising a plurality of layers, each layer comprising a plurality of rows, each row comprising one volume-building component. Fig. 1(f) is a side view of the cylindrical scaffold 1 shown in fig. 1(e), comprising nine layers 3 (a so-called 9-layer stack), and wherein H is the height of the scaffold.

It is to be understood that the phrase "layer extends in an x-y-plane" as used herein may be interpreted as "layer extends *essentially* in an x-y-plane", meaning that any layer of a scaffold is able to extend in an x-y-plane but does not necessarily have to be completely flat. The scaffold material itself may have certain flexibility and softness, to make the scaffold adaptable to the forms of surrounding tissue when implanted in an individual. Additionally or alternatively, the scaffold may be printed on top of a soft textile or mesh and included in a medical implant which should likewise be formable in surrounding tissue in the body. The terms "x-y-plane" and "horisontal plane" may be used interchangeably herein.

Similarly, the phrase "layer has a height (H) in a z-direction perpendicular to the x-y-plane" as used herein may be interpreted as "layer has a height (H) in a z-direction *essentially* perpendicular to the x-y-plane", meaning that the height of a layer is normally measured in a z-direction perpendicular to the x-y-plane, but the height of a layer does not necessarily have to be completely perpendicular to the x-y-plane. Again, this is due to the fact that the scaffold material itself may have certain flexibility and softness.

Herein, the term "volume-building component" includes any component which is capable of adding to the volume of the scaffold. A volume-building component may take any suitable form, and preferably has an elongated shape or form, such as a strand. Further, herein a volume-building component may comprise a plurality of volume-building elements joined together, provided that an element has a smaller size than a component. As a non-limiting example, a volume-building element may have the form of a droplet or sphere, and a plurality of such droplets or spheres may be joined together to form a strand. Some types of apparatuses for additive manufacturing are configured to deposit scaffold material in the form of elongated volume-building components, such as strands. In contrast, other types of apparatuses for additive manufacturing are configured to deposit scaffold material in the form of spheric volume-building elements, such as droplets or spheres, which may be adhered or fused together to form a chain of a predefined number of volume-building elements. The volume-building elements may for example be adhered or fused together by use of a suitable temperature, which is higher than the melting temperature of the scaffold material, while depositing the volume-building elements. Such a chain of volume-building elements may correspond to an elongated volume-building component in terms of its properties. The length of each chain of volume-building elements may be controlled. The chains of volume-building elements may be distributed in any desired pattern within one layer, such as a staggered distribution. Further, the distance Dx between any two adjacent chains of volume-building elements of a first group of layers may be controlled and may be different from the distance Dx between any two adjacent chains of volume-building elements of a second group of layers which is adjacent to the first group of layers.

In the context of the volume-building components being aligned in rows in the x-y-plane of any layer of the presently disclosed scaffold, the term "aligned" is intended to mean that essentially all volume-building components of a layer are arranged or positioned essentially in the same direction(s) or shape(s) in the x-y plane, but not necessarily in straight lines, meaning that the rows do not necessarily have to be straight lines. The volume-building components may for example be curved or otherwise shaped in the x-y-plane, resulting in rows having a curved or other shape. However, it is contemplated that essentially all volume-building components of a layer may be arranged in parallel to each other, meaning that all rows of a layer would be parallel to each other.

Throughout the present text, the term "adjacent" is defined as meaning next to, but not necessarily joined with, something else. Two components of the same type which are adjacent to each other do not have any component of the same type positioned inbetween them but may have a component of another type positioned inbetween them. For example, according to the present disclosure a first volume-building component and a second volume-building component which are adjacent (in a layer, or in a row) are positioned next to each other, without any third volume-building component positioned inbetween the first and second volume-building components. However, the first volume-building component and the second volume-building component normally have a certain distance between them in the x-direction and/or in the y-direction of the layer. According to another example of the present disclosure, a first layer and a second layer which are adjacent to each other are positioned next to each other in the scaffold, without any third layer positioned inbetween the first and second layers. Two adjacent layers may be joined (such as adhered, e.g. fused) or alternatively, therer may be another type of component positioned inbetween them, in which case there is a certain distance between them in the z-direction. In the context of the present disclosure it is contemplated that for example a mesh or any type of supporting component may be positioned between any two adjacent layers of volume-building components. Thereby, the structure of the scaffold may be altered in many ways, which in turn results in different mechanical properties of the scaffold. In applications where a pliable scaffold is desirable, at least some of the layers of a scaffold preferably do not adhere to any adjacent layer, more preferably none of the layers adhere to any adjacent layer. In contrast, in applications where a scaffold with high rigidity is sought for, at least some of the layers of a scaffold, such as all layers, suitably adhere to adjacent layers, since adherence will increase the strength of the scaffold.

Herein, the term "group of volume-building components" is defined as being a group comprising at least one volume-building component, such as 1, 2, 3, 4, 5, 6, 7, 8, 9, 10 or more volume-building components. In an embodiment where the group comprises more than one volume-building component, the volume-building components of the group are adjacent to each other.

The term "group of layers" is to be interpreted as being a group comprising at least one layer, such as 1, 2, 3, 4, 5 or more layers. In an embodiment where the group comprises more than one layer, the layers of the group are adjacent to each other.

The term "group of rows" is to be interpreted as being a group comprising at least one row, such as 1, 2, 3, 4, 5 or more rows. In an embodiment where the group comprises more than one row, the rows of the group are adjacent to each other.

The m volume-building components of a any group of layers are distributed or oriented at an angle alpha ($\alpha$) in the x-y-plane relative to the m volume-building components of any adjacent group of layers. In other words, the plurality of volume-building components of a first group of layers are aligned and are positioned or distributed or oriented at an angle $\alpha$ relative to the plurality of volume-building components of a second group of layers adjacent to the first group of layers. In this context, it is to be understood that a group of layers may suitably consist of one layer only, or alternatively may suitably consist of two layers. However, for some applications it is envisioned that it may be suitable to make a group consisting of more than two layers adjacent to each other have the same angle of orientation in relation to an adjacent group of layers. Further, the term "at an angle $\alpha$ in the x-y-plane" may alternatively be expressed as "at an angle $\alpha$ in a z-axis view". Preferably said angle alpha is in a range of from >0° to 90°, such as 0.1°, 0.5°, 1°, 5°, 10°, 15°, 20°, 25°, 30°, 35°, 40°, 45°, 50°, 55°, 60°, 65°, 70°, 75°, 80°, 85°, or 90°. Changing the angle $\alpha$ results in a scaffold having a different stress response. For example, an angle $\alpha$ of 15° leads to a better stress distribution within the scaffold, and thus the scaffold has a lower stiffness compared to an angle $\alpha$ of 90°. It is to be understood that different degrees of the angle $\alpha$ are suitable for different applications in the field of medical technology.

The scaffold comprises a gradient distribution of volume-building components in the z-direction, wherein the distance Dx between any two adjacent groups of volume-building components of any group of layers is different from the distance Dx between any two adjacent groups of volume-building components of any adjacent group of layers. In other words, the distance Dx between (any two) adjacent groups of volume-building components of a first group of layers is different from the distance Dx between (any two) adjacent groups of volume-building components of a second group of layers which is adjacent to the first group of layers.

The gradient distribution of volume-building components in the z-direction may be achieved for example by any one or a combination of the following presently preferred configurations:

(a) The distance (Dx) between any two adjacent groups of volume-building components of a group of layers at a first end portion in the z-direction and/or at a second end portion in the z-direction of the scaffold is larger than the distance (Dx) between any two adjacent groups of volume-building components of a group of layers at an intermediate portion in the z-direction of the scaffold. This configuration results in scaffolds having a smaller pore size in the middle than at one or both ends in the z-direction of the scaffold. It is to be understood that this configuration may also include a repetitive pattern of alternating pore sizes in the z-direction of the scaffold.

(b) The distance (Dx) between any two adjacent groups of volume-building components of a group of layers at a first end portion in the z-direction of the scaffold is larger than the distance (Dx) between any two adjacent groups of volume-building components of a group of layers at a second end portion in the z-direction of the scaffold. This configuration results in scaffolds having a smaller pore size at one end and a larger pore size at the other end in the z-direction of the scaffold, or in other words results in scaffolds having a gradient of pore sizes from one end to the other end in the z-direction of the scaffold.

A third, currently less preferred, configuration is also contemplated, including a smaller pore size at both end portions than at an intermediate portion in the z-direction of the scaffold. It is to be understood that this configuration may also include a repetitive pattern of alternating pore sizes in the z-direction of the scaffold, meaning alternating decreased pore size and increased pore size, repeated periodically.

The minimum number of layers to achieve a gradient distribution of pore sizes in a scaffold is 4 layers, since a minimum of 2 layers are needed to form one layer of pores with a specific size, and thus 4 layers are required to form a gradient. Apart from this limitation in terms of a minimum number of layers to achieve a gradient distribution, it is to be understood that the properties of a scaffold are not dependent upon the number of layers in the scaffold. Suitably, the intended site or medical application of a scaffold may decide how many layers a specific scaffold should have.

Each volume-building component has a length L. The length L of each volume-building component affects the mechanical resistance to a deformation in the x-y-plane of a scaffold having a staggered distribution, and consequently also affects the properties of a medical implant comprising such a scaffold. For instance, the staggered structure results in a lower tensile modulus in the x-y-plane compared to a non-staggered structure. This length L may alternatively be called "continuous length" in the art. The length L depends on the intended site and medical application of a specific scaffold, and may vary within one scaffold. Volume-building components used in scaffolds described herein may suitably have a length L in a range of from about 1 mm to about 300 mm.

The height and width of a volume-building component may suitably be in a range of from about 0.05 mm to about 3 mm.

Further, the length or gap or distance between two adjacent volume-building components positioned in the same row or in adjacent rows of a layer also affects the mechanical resistance of a scaffold having a staggered distribution in a similar way as described above. This length or gap or distance may alternatively be denoted by the term "discontinuous length".

Throughout the present text, the term "discontinuous length" is distinguished as a distance Dx in the x-direction when describing the distance between a volume-building component in any row of a layer and any adjacent volume-building component in any adjacent row of the same layer. Further, it is to be understood that in the phrase "the distance Dx between any two adjacent groups of volume-building components of any group of layers", the distance Dx referred to is the distance Dx between a first volume-building component belonging to a first group of volume-building components, which first volume-building component is located at an edge of said first group, i.e. outermost in said first group, and which first volume-building component is located closest to a second volume-building component belonging to a second group of volume-building components, which second volume-building component is located outermost in said second group at an edge of said second group, which edge is located closest to said first group. The distance Dx depends on the intended site and medical application of a specific scaffold, and will vary within one scaffold to achieve a gradient distribution. However, normally the distance Dx may suitably be in a range of from about 0.2 $\mu$m to about 1500 $\mu$m.

Further, when describing the distance between a volume-building component in any row of a layer and any adjacent volume-building component in the same row, the term "discontinuous length" is distinguished as a distance Dysr (wherein "sr" stands for same row) in the y-direction. The distance Dysr depends on the intended site and medical application of a specific scaffold, and may vary within a scaffold. However, normally the distance Dysr may suitably be in a range of from about 0.2 $\mu$m to about 1500 $\mu$m.

Additionally, when describing the distance between a volume-building component in any row of a layer and any adjacent volume-building component in an adjacent row of the same layer, the term "discontinuous length" is distinguished as a distance Dyar (wherein "ar" stands for adjacent rows) in the y-direction. Further, it is to be understood that in the phrase "each group of volume-building components in any group of rows of a layer is positioned at a distance Dyar in the y-direction from any adjacent group of volume-building components in any adjacent group of rows of the same layer", the distance Dyar referred to is the distance Dyar between a first volume-building component belonging to a first group of rows of a layer, which first volume-building component is located at an edge of said first group, i.e. outermost in said first group, and which first volume-building component is located closest to a second volume-building component belonging to a second group of rows of the same layer, which second volume-building component is located outermost in said second group at an edge of said second group, which edge is located closest to said first group. The distance Dyar depends on the intended site and medical application of a specific scaffold, and may vary within a scaffold. However, normally the distance Dyar may suitably be in a range of from about 0.2 $\mu$m to about 1500 $\mu$m.

The scaffold comprises a staggered distribution of volume-building components in the x-y-plane, wherein each volume-building component in any row of a layer is positioned at a distance (Dysr) in the y-direction from any adjacent volume-building component in the same row, and wherein each group of volume-building components in any group of rows of a layer is positioned at a distance (Dyar) in the y-direction from any adjacent group of volume-building components in any adjacent group of rows of the same layer. In other words, the horizontal position of a group of volume-building components of a layer is offset relative to the horizontal position of any adjacent group of volume-building components of said layer.

It should be clarified that a staggered distribution of volume-building components in a scaffold can be achieved through different configurations in terms of 'discontinuous length'. For example, a scaffold comprises only Dysr, or Dyar, or a combination of Dysr and Dyar to form a staggered distribution of volume-building components. In this regard, the minimum number of volume-building components at one layer to achieve a staggered distribution in a scaffold is varying. Specifically, a minimum of 2 volume-building components are needed to form an offset (Dyar) from one row to another at one layer and a minimum of 4 volume-building components are needed to form an offset (Dysr) from one row to another, wherein there are at least 2 volume-building components in one row.

More particularly, the staggered distribution of volume-building components, wherein the horizontal position of a group of volume-building components of a layer is offset relative to the horizontal position of any adjacent group of volume-building components of said layer, may be achieved for example if the length L of each of a group of volume-building components of a layer is smaller than the width W of said layer, i.e. L < Wx or L < Wy. Alternatively, or additionally, the staggered distribution may be achieved, even if L > Wx or L > Wy, if each of a group of volume-building components of a layer is distributed, positioned or formed, e.g. curved or diagonally positioned, within the width Wx and/or Wy of the layer, such that each of the group of volume-building components extends a distance in the x-direction and/or a distance in the y-direction, which distance is shorter than the width Wx and Wy, respectively, of said layer.

Each layer of a scaffold has a height H in the z-direction, i.e. along a z-axis. The height H of each layer may be the same for all layers of a scaffold or may be different for different layers of a scaffold. The height of a layer depends on the intended site and medical application of the scaffold, and may vary within a scaffold. However, normally the height H may suitably be in a range of from 0.05 to 0.4 mm.

The height of a complete scaffold, comprising a plurality of layers, depends on the intended site and medical application of the scaffold. However, normally the height of a complete scaffold may suitably be in a range of from about 0.2 mm to about 10 mm. In a currently preferred medical application of the scaffold, namely reconstruction of a breast, the height of a scaffold may preferably be from about 0.2 mm to about 3 mm, wherein the maximum height corresponds to the thickness of the so-called Cooper's ligament in the breast.

Each layer of a scaffold may be defined as having a width Wx in the x-direction and a width Wy in the y-direction. The width Wx of a layer may be equal to the width Wy of the layer, in which case the layer has a circular shape. Alternatively, the width Wx of a layer may be different from the width Wy of the layer, resulting in any type of shape of the layer. Further, it is to be understood that different layers of a scaffold may have different widths and different shapes, depending on the intended application in each specific case, for example the type and/or shape of a defect to be treated, or the type of tissue to be treated. The widths Wx and Wy of a layer may suitably be in a range of from about 1 mm to about 300 mm.

The scaffold according to the present disclosure may comprise scaffold material which is resorbable or degradable *in vivo.*

The scaffold material used to prepare the scaffold according to the present disclosure may comprise a polymer, a copolymer or a combination of different polymers and/or copolymers. Preferably, the polymer(s) and/or copolymer(s) comprise one or more monomers selected from a group consisting of glycolide, lactide (D- or L-, meso or racemic mixture), trimethylene carbonate, ε-caprolactone, p-dioxanone (i.e. paradioxanone), β-butyrolactone and 1,5-dioxepan-2-one, or combinations thereof. More preferably, the scaffold material comprises a copolymer of ε-caprolactone and p-dioxanone as disclosed herein. Such a copolymer preferably has a random distribution of ε-caprolactone and p-dioxanone units along the polymeric chain and a higher content of ε-caprolactone than p-dioxanone. The copolymer is prepared by bulk ring-opening polymerization of a mixture of the two monomers and has preferably a melting poing between 60 °C and 25 °C. The copolymer forms films with a Young's Modulus between 50 and 400 MPa and elongation to break between to 200 and 2000 %, and hydrolytic degradation time between 8-24 months depending on the composition, but much faster than PCL.

The microstructure of a polymer or copolymer, e.g. random or blocky microstructure, influences the physical properties of the scaffold. Thus, the microstructure is one parameter which may be varied to achieve scaffolds which are suitable for different medical applications. It is to be understood that for the purpose of preparing a scaffold by additive manufacturing according to the presently disclosed method, the microstructure of the polymer may be of any type, such as random, or blocky, as long as the parameters of the method for preparing the scaffold are tailored to the type of microstructure desired for each medical application.

The present disclosure further provides a medical device for use in tissue engineering, comprising a scaffold as described in detail above.

The medical device may further comprise any suitable type of support material, such as a non-woven or mesh material, for example in the form of a sheet of mesh, or a homogeneous film.

Alternatively or in addition, the medical device as described above may comprise a plurality of scaffolds as described above, wherein the plurality may comprise 2, 3, 4, 5, 10, 15, 20 or more scaffolds. Such scaffolds included in the same medical implant may have identical or different properties. As a non-limiting example, the porosity of two scaffolds within the same medical implant may be identical or different. Other properties which may vary between scaffolds within a medical implant are the pore size, pore structure, interconnectivity (i.e. open porosity), and mechanical properties.

It is to be understood that the presently disclosed scaffold, as well as the presently disclosed medical device, are preferably configured to be implanted in an individual. Non-limiting examples include that the scaffold or medical device may be configured to be sutured or stapled or otherwise fastened to surrounding tissue, and/or may be pliable to conform to the surrounding tissue.

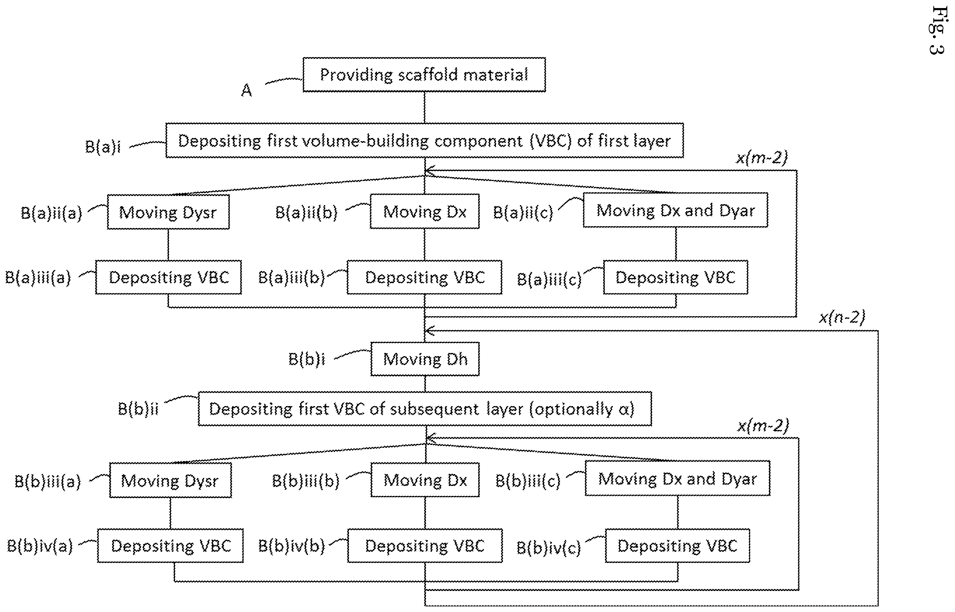

The present disclosure further provides a method for preparing a scaffold, as illustrated schematically in the flow chart of Fig. 3, wherein the scaffold is configured for use in tissue engineering, the scaffold comprising n layers extending in an x-y-plane, where n is an integer $\geq 4$, each layer having a height (H) in a z-direction, and each layer comprising m volume-building components aligned in r rows in the x-y-plane, where m is an integer $\geq 2$, where r is an integer $\geq 2$; the method comprising:

(A) providing a scaffold material;
(B) preparing a scaffold from the scaffold material in an apparatus configured for additive manufacturing comprising a material source configured to deposit volume-building components of scaffold material, comprising:

(a) forming a first layer of the scaffold on top of a base plate of the apparatus configured for additive manufacturing, comprising:

i. depositing a first volume-building component of the scaffold material at a position on top of the base plate;
ii. moving the material source:

   a. a distance (Dysr) in the y-direction, or
   b. a distance (Dx) in the x-direction, or
   c. a distance (Dx) in the x-direction and a distance (Dyar) in the y-direction;

iii. depositing a subsequent volume-building component of the scaffold material at a subsequent position on the base plate, wherein the subsequent position is located:

   a. at a distance (Dysr) in the y-direction from the position of the preceding volume-building component deposited, meaning that the subsequent volume-building component is in the same row as the preceding volume-building component, or
   b. at a distance (Dx) in the x-direction from the position of the preceding volume-building component deposited, and wherein the subsequent volume-building component is aligned in the x-y-plane with the preceding volume-building component deposited, meaning that the subsequent volume-building component is positioned in a subsequent row but at the same position in the y-direction as the preceding volume-building component, i.e. the two volume-building components being part of a group of volume-building components in adjacent rows, or
   c. at a distance (Dx) in the x-direction and at a distance (Dyar) in the y-direction from the position of the preceding volume-building component deposited, and wherein the subsequent volume-building component is aligned in the x-y-plane with the preceding volume-building component deposited, meaning that the subsequent volume-building component is positioned in a subsequent row at a different position in the y-direction compared to the preceding volume-building component;

iv. repeating steps ii-iii (m-2) times, to deposit the m volume-building components of the first layer;

(b) forming (n-1) subsequent layers on top of the first layer of the scaffold in the apparatus configured for additive manufacturing, comprising:

i. moving the material source a distance (Dh) in the z-direction, equal to the height (H) of a layer;
ii. depositing a first volume-building component of the scaffold material at a position on top of the preceding layer, optionally at an angle ($\alpha$) in the x-y-plane relative to the position of the first volume-building component of the preceding layer;
iii. moving the material source:

   a. a distance (Dysr) in the y-direction, or
   b. a distance (Dx) in the x-direction, or
   c. a distance (Dx) in the x-direction and a distance (Dyar) in the y-direction;

iv. depositing a subsequent volume-building component of the scaffold material at a subsequent position on top of the preceding layer, wherein the subsequent position is located:

   a. at a distance (Dysr) in the y-direction from the position of the preceding volume-building component deposited, or
   b. at a distance (Dx) in the x-direction from the position of the preceding volume-building component deposited, and wherein the subsequent volume-building component is aligned in the x-y-plane with the preceding volume-building component deposited, or
   c. at a distance (Dx) in the x-direction and at a distance (Dyar) in the y-direction from the position of the preceding volume-building component deposited, and wherein the subsequent volume-building component is aligned in the x-y-plane with the preceding volume-building component deposited;

v. repeating steps iii-iv (m-2) times, to deposit the m volume-building components of any subsequent layer;
vi. repeating steps i-v (n-2) times, to form the n layers of the scaffold;

   provided that the distance (Dx) in any group of layers is different from the distance (Dx) of the preceding group of layers;
   provided that the m volume-building components of any group of layers are distributed at an angle ($\alpha$) in the x-y-plane relative to the m volume-building components of any adjacent group of layers;

thereby preparing a scaffold comprising a gradient distribution of volume-building components in the z-direction of the scaffold, and a staggered distribution of volume-building components in the x-y-plane of the scaffold;

wherein the height (H), the distance (Dx), the distance (Dysr), the distance (Dyar) and the angle ($\alpha$) together define (or determine) a size and shape of pores in the scaffold.

It is to be understood that by performing the above-described method, each volume-building component in any row of a layer of the scaffold will be positioned at a distance (Dx) in the x-direction from any volume-building component in any adjacent row of the same layer. Further, the m volume-building components of any group of layers will be distributed at an angle ($\alpha$) in the x-y-plane relative to the m volume-building components of any adjacent group of layers. Also, the scaffold will comprise a gradient distribution of volume-building components in the z-direction, wherein the distance (Dx) between any two adjacent groups of volume-building components of any group of layers is different from the distance (Dx) between any two adjacent groups of volume-building components of any adjacent group of layers, and the scaffold will comprise a staggered distribution of volume-building components of a layer in the x-y-plane, wherein each volume-building component in any row of a layer is positioned at a distance (Dysr) in the y direction from any adjacent volume-building component in the same row, and wherein each group of volume-building components in any group of rows of a layer is positioned at a distance (Dyar) in the y-direction from any adjacent group of volume-building components in any adjacent group of rows of the same layer.

The term "apparatus configured for additive manufacturing" is intended to mean an apparatus configured to build layers of volume-building components of scaffold material. Additive manufacturing uses data computer-aided-design (CAD) software or 3D object scanners to direct hardware to deposit material, layer upon layer, in precise geometric shapes. Such an apparatus may alternatively be called for example a rapid prototyper, a manufacturing processer, or a 3D printer. The apparatus comprises a base plate and a material source. The term "material source" may alternatively be called head of a material feeding system, head of material extrusion system, nozzle, or printer head. It is to be understood that different types of apparatuses configured for additive manufacturing may be used to perform the presently disclosed method for preparing a scaffold. Non-limiting examples of suitable apparatus for use in the presently disclosed method are apparatus configured for filament extrusion and fused deposition modeling (FDM), fused filament fabrication (FFF), 3D dispensing, 3D bioplotting extrusion and material jetting (such as freeformer), or Directed Energy Deposition (DED).

Further, the person skilled in the art understands that parameters and conditions suitable to apply in the method for preparing a scaffold, e.g. time, temperature, dimensions of volume-building components, distances Dx, Dysr, Dyar and height **H,** will vary depending on the intended site or medical application for a specific scaffold. Dimensions of volume-building components, distances Dx, Dysr, Dyar and height H have been described in detail above. Further below is given two non-limiting examples of a method for preparing a scaffold comprising a presently preferred scaffold material.

The distance Dyar between two adjacent volume-building components, positioned in two adjacent rows of a layer, may be as long as the entire width Wx or Wy of the layer, meaning that after the material source has deposited the last volume-building component of a row, the material source moves back the entire width Wx or Wy of the layer before starting to deposit the first volume-building component of the subsequent row.

It is common knowledge in the art that in many apparatuses configured for additive manufacturing, either the material source, the base plate or both of them may be moved. This means that instead of moving the material source a certain distance Dx, Dysr, and/or Dyar before depositing a subsequent volume-building component or moving the material source a distance (Dh) in the z-direction, equal to the height (H) of a layer, before forming a nsubsequent layer, the base plate may be moved an equal distance in the opposite direction to achieve the same structure of the scaffold.

As mentioned above in relation to the presently disclosed scaffold, adjacent layers may be made to adhere to each other but not necessarily. Conditions sufficient to adhere a subsequent layer to the preceding layer will vary depending on for exmple the type of scaffold material used.

The scaffold material used in the presently disclosed method may comprise a polymer, a copolymer or a combination of different polymers and/or copolymers, preferably wherein the polymer(s) and/or copolymer(s) comprise one or more monomers selected from a group consisting of glycolide, lactide (D- or L-, meso or racemic mixture), trimethylene carbonate, $\epsilon$-caprolactone, p-dioxanone, $\beta$-butyrolactone and 1,5-dioxepan-2-one, or combinations thereof, more preferably wherein the scaffold material comprises a copolymer of $\epsilon$-caprolactone and p-dioxanone. The scaffold material used preferably has a melting point below or equal to 60 °C and a thermal degradation starting 200 °C above the melting point. Such material preferably is a random copolymer of $\epsilon$-caprolactone and p-dioxanone and has higher content of $\epsilon$-caprolactone than p-dioxanone.

When using the presently preferred scaffold material, i.e. comprising a copolymer of $\epsilon$-caprolactone and p-dioxanone, when performing the method for preparing a scaffold, the method may comprise the following non-limiting conditions. Fused deposition modeling (FDM) at temperature between 140-220 °C and speed 10-20 mm s$^{-1}$ of extruded filament of the copolymer at 125 °C and 9 rpm can be used for scaffold preparation, or 3D bioplotting of polymer pellets at temperature in the range 140-160 °C and 8.5 bar of pressure.

According to yet another aspect, the present disclosure provides a method for *in vivo* tissue engineering comprising implanting the presently disclosed scaffold or medical device into an individual. In particular, such a method may be applied for soft tissue engineering, such as adipose tissue engineering, and more particularly for breast reconstruction, such as after mastectomy, wherein the scaffold may preferably be used for supporting a breast prosthesis. Without wishing to be bound by theory, it is believed that the scaffold may be configured to mimic the so-called Cooper's ligament which is a supporting structure, like a frame, in the mammalian breast. Other specific applications of such a method for *in vivo* tissue engineering include reconstruction of tendon, ligament or muscle junction in an individual.

Further provided is the presently disclosed scaffold for use in a method for *in vivo* tissue engineering as described in detail above. Also provided is the presently disclosed medical device for use in a method for *in vivo* tissue engineering as described in detail above.

The present disclosure is also directed to in vitro uses of the presently diclosed scaffold, such as, but not limited to, a use of the presently diclosed scaffold in an *in vitro* cell culture system, in an *in vitro* method for culturing of cells and/or in an *in vitro* method for regenerating tissue.

Herein, devices and scaffolds "comprising" one or more recited elements may also include other elements not specifically recited. The term "comprising" includes as a subset "consisting essentially of" which means that the device or scaffold has the components listed without other features or components being present. Likewise, methods "comprising" one or more recited steps may also include other steps not specifically recited. Further, the singular "a" and "an" shall be construed as including also the plural.

*Experimental section*

**Example 1: Computational and experimental characterizations of designed and 3D printed PCL scaffolds for soft tissue engineering**

**1. Introduction**

For a scaffold with a given porosity, the mechanical properties can be tailored by altering the strand alignments. Moreover, internal structure has a great impact on crucial factors for soft tissue regeneration, such as transport of nutrient, adhesion of cells and deposition of matrix. A computer-aided design method with the introduction of computational simulation technologies, namely finite element analysis (FEA) and computational fluid dynamics (CFD), is suitable to gain a priori knowledge of a scaffold architecture in terms of mechanical properties and transport behaviors. A further step is to fabricate designed 3D scaffolds and validate the computational analysis against experimental results. In this study, FDM technology was utilized to fabricate scaffolds with present designs and thereafter validate numerical analysis. During printing, filaments are melted through the heated extruder and deposited on the printer bed layer by layer to create an object. This method is the most cost-effective way of producing thermoplastic objects and prototypes. The objective of this study was to investigate the effect of designed structures on the mechanical properties and transport behaviors of 3D porous scaffolds by means of numerical and experimental procedures.

**2. Materials and Methods**

**2.1 Scaffold structure design**

In this study, ten 3D structural designs are presented in Table 1. For all designs, a 9-layer scaffold was constructed by repeating strands with a constant thickness of 0.4 mm. The difference in scaffold designs were controlled by tailoring strand orientation (SO), strand space (SS) and strand thickness (ST). The original length of each strand was 12 mm. After assigning SO, SS and ST values to a given design, a boolean operation was carried out to obtain a scaffold with a cylindrical shape. The radius was 5 mm. To be specific, three types of structures were examined, namely normal (N), gradient (G) and gradient + stagger (GS). A normal structure is referred to as SO changes from one layer to another while keeping a constant SS at the same layer. Five different SO values of 10, 15, 30, 45 and 90 degree combines with a constant SS value of 0.4 mm (0.8 and 1.2 mm for 90 degree as well) were considered for a normal structure. Moreover, a varying SS was presented in a gradient structure, which possesses a large SS at the first end and the second end portion and a small SS at the middle portion in the perpendicular direction of a scaffold. Two SO values of 15 and 90 degree are combined with a varying SS value of 1.2-0.8-0.4-0.8-1.2 from the first end to the second end of a scaffold. Furthermore, a combination of a staggered structure and a gradient structure was formulated. In the staggered structure, a shorter length of 8 mm was assigned to each strand. Meanwhile, strands were aligned in a staggered manner by offsetting their horizontal positions to adjacent strands. As a consequence, strands at each layer were separated from the boundary of the scaffold with a certain distance. Only one SO value of 15 degree combines with a varying SS values of 1.2-0.8-0.4-0.8-1.2 mm for gradient + stagger structure. The theoretical porosity of each scaffold is listed in Table 1, which is estimated by following equation,

$$p = (1 - V_{solid})/V_{total} \qquad (1)$$

where p is the porosity, $V_{solid}$ is the solid volume of a scaffold structure and $V_{total}$ is total volume of the occupied by a scaffold structure.

## 2.2 Computational analysis

The 3D model was built up in ABAQUS 6.14 and used for FEA and CFD simulations (Fig. 1). In FEA model, a 3D scaffold was placed between two rigid plates for both compression and tension model. Material properties of poly($\varepsilon$-caprolactone) (PCL) filament were assigned for scaffolds. The elastic modulus of 345 MPa for PCL was used. Boundary conditions were defined at these two rigid plates. For the compression model, the bottom plate is fixed and the top plate is displaced to an axial strain of 1% to simulate the compression. For the tension model, a 9-layer scaffold with a rectangle shape was considered with a dimension of 9.8 mm in both length and width. The tension was simulated by applying an axial strain of 0.5 % for both the left and the right plate. The reaction force at the rigid plate was recorded from simulations and used to calculate the effective compressive/tensile modulus from following equation,

$$E = F_R/(A\varepsilon) \qquad (2)$$

where E is the effective compressive/tensile modulus, $F_R$ is the reaction force, A is the cross sectional area, and $\varepsilon$ is the strain.

In CFD model, the scaffold was inserted into a virtual bioreactor that includes one inlet and one outlet. The velocity and the pressure throughout the model were obtained by solving the continuity and Navier-Stokes equations using ABAQUS CFD solver. Water was considered as the working fluid, which has a density of 1000 kg·m$^{-3}$ and a dynamic viscosity of 0.001 kg·m$^{-1}$·s$^{-1}$. The diameter of the inlet and the outlet was 0.002 m. A velocity of 10$^{-4}$ m·s$^{-1}$ was applied for the inlet, which guarantees Reynolds number Re < 1 in the range of validity of Darcy's law. A zero pressure was defined as the boundary condition at the outlet. Moreover, no-slip wall conditions were assigned for the walls of the scaffold and the virtual bioreactor. The average pressure values at the top and the bottom of the scaffold were extracted and used to determine of the permeability coefficient from the Darcy's law,

$$k = Q\mu L/(A\Delta P) \qquad (3)$$

where k is the permeability, Q is the fluid flow rate, L is the length of the scaffold, A is the cross sectional area, and $\Delta P$ is the pressure drop from the top to the bottom of the scaffold.

Thereafter, all the permeability coefficient obtained were normalized by the reference permeability coefficient, which is predicted from a separate CFD analysis of a 100% porosity model.

## 2.3 Fabrication of PCL scaffolds

Scaffolds were printed using the fifth generation MarkerBot Replicator Desktop 3D Printer (Stratasys, USA). The 3D printer uses filaments of 1.75 mm in diameter and it was fitted with a nozzle of 0.40 mm. All CAD files were made in Abaqus then sliced and converted to .makerbot file using MakerBot Print. The PCL filaments (wt.%) used were commercially available (3D4Makers, Netherlands) and used as received. The slicing distance was set to 0.4 mm and the printing speed to 10 mm$^{-1}$. No infill, roof, floors, rafts or additional support structures were used. The retraction distance was set to zero and one shell was used, of each scaffold designs ten structures were produced each time each time at a printing temperature of 100 °C with no additional cooling of the printing plate, the excess polymer was cut away from the structures.

**Table 1.** Description of structural designs for scaffolds.

| Type⧸Design | N | | G | GS |
|---|---|---|---|---|
| | | | | |

| SO, degree | 10 | 15 | 30 | 45 | 90 | 90a | 90b | 15 | 90 | 15 |
|---|---|---|---|---|---|---|---|---|---|---|
| Top view | Fig. 2(a) | Fig. 2(b) | Fig. 2(c) | Fig. 2(d) | Fig. 2(e) | Fig. 2(f) | Fig. 2(g) | Fig. 2(h) | Fig. 2(i) | Fig. 2(j) |
| Side view of mid-cross section | Fig. 2(k) | Fig. 2(l) | Fig. 2(m) | Fig. 2(n) | Fig. 2(o) | Fig. 2(p) | Fig. 2(q) | Fig. 2(r) | Fig. 2(s) | Fig. 2(t) |
| ST, mm | 0.4 | | | | | | | | | |
| Number of layers | 9 | | | | | | | | | |
| SS, mm | 0.4 | | | | | 0.8 | 1.2 | 1.2/1.2/0.8/0.8/0.4/ 0.8/0.8/1.2/1.2 | | |
| Porosity, % | 60.4 | | | | | 73.5 | 77.9 | 75 | | 79.7 |

**2.4** Mechanical testing

Compressive testing of printed scaffolds (N15, N90, G90, G15 and GS15) were conducted to determine mechanical properties using the Instron 5566 tensile testing instrument at room temperature and 50% humidity using a 10 kN load cell after have been equilibrated for 24 hours. The scaffolds were compressed at a deformation speed of 0.35 mm$^{-1}$, the equivalent of approximately 10 % of the height of the scaffolds. The force and the displacement were recorded throughout the testing, and thereafter converted to a stress-strain curve. Each scaffold was compressed at least 1.5 mm and the compressive modulus reported for scaffolds was defined as the slope of the initial linear region in the stress-strain curve (n=7).

**2.5 Microcomputed tomography ($\mu$-CT) characterization of scaffolds**

All scaffolds were scanned using Skyscan 1172 system (Bruker Micro-CT, Kontich, Belgium) at 9 $\mu$m spatial resolution. For all scans, the operation voltage setting was 40 kV, the current utilized was 250 $\mu$A, and no filter was employed. A step size of 0.4° and frame averaging of 2. For reconstructions, NRecon (Bruker Micro CT, Kontich, Belgium) were carried out using full cone beam Feldkamp reconstruction algorithm with greyscale limits defined automatically (Need to insert REF -check Mohammeds paper). The microstructure of 3D printed scaffolds of PCL were analyzed from the CT images using CTAn (CT analyser, ver. 1.18, SkyScan). For each scaffold, region of interest (ROI) was selected and thresholding of these ROI was carried out using automatic Otsu thresholding. Surface area, total porosity, interconnectivity, mean pore size and pore size distribution were identified (n=3). 3D images were generated using CTVox (CTVox version 2.7, SkyScan) software

**3. Results and Discussion**

**3.1 Mechanical properties**

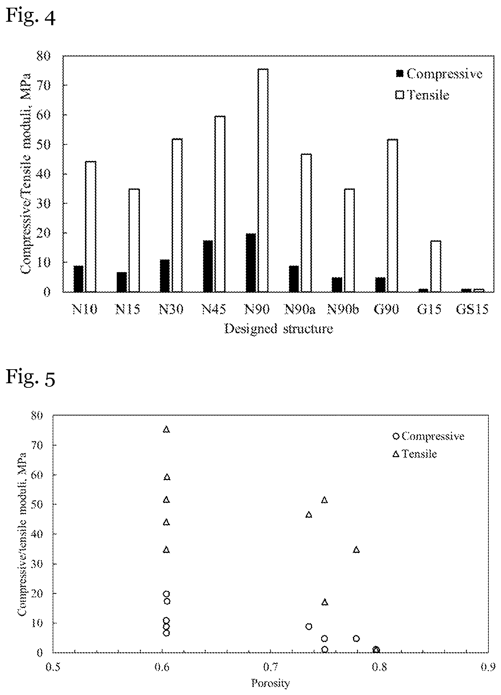

The compressive and tensile moduli of five designed structure obtained from FEA simulations are shown in Fig. 4. It can be obviously observed that the gradient and gradient + stagger structural designs with a given strand orientation lead to a much softer scaffold with rather low effective compressive/tensile moduli compared to the normal structure. This can be explained by the fact that the gradient and gradient + stagger structures possess higher porosity than the normal structure. As a result, the resistance of the scaffold structure with a low volume fraction in solid to the mechanical stimulation is reduced. Likewise, a larger strand space gives N90a and N90b scaffolds a higher porosity compared to N90 scaffold, which results in a lower effective compressive/tensile moduli. However, there is also evidence for designs with the same type of structure, which indicates that mechanical properties of scaffolds are independent of porosity effect. For instance, designs with a normal structure have the same theoretical porosity, whereas their compressive/tensile moduli show a clear difference from N10 to N90. Specially, N15 scaffold shows the lowest effective compressive/tensile modulus. Similarly, the effective compressive/tensile modulus of G15 is smaller than that of G90. The relation between the porosity and the effective compressive/tensile moduli for all studied scaffolds is summarized in Fig. 5. It clearly shows that the difference in mechanical responses of a scaffold to compression/tension can be attributed to the effect of the structure. Moreover, stress concentrations were observed at the regions where layers were connected. It indicates that mechanical responses depend on how strands align within a scaffold to a given loading condition. This, in turn, decides the extent to which a scaffold deforms under mechanical stimulations. Specifically, strand orientation shows a clear influence on the stress distribution for scaffolds with the normal structure. Also, the gradient and gradient + stagger structural designs distribute the stress within the scaffold in a better way, which result in fewer regions occupied by high-stress values. Furthermore, computational analysis shows that GS15 scaffold has the lowest compressive/tensile modulus, 1.07/0.97 MPa. It is remarkable that the mechanical properties of GS15 scaffold are as low as 0.23% of that of pure PCL filament. It is worth noting that the tensile modulus of scaffolds with a strand orientation of 15 degree can dramatically be decreased from 17.22 MPa (G15) to 0.97 MPa (GS15) by introducing a stagger design structure. The reason is that the staggered structure has fewer strands in the horizontal plane along with the load direction (tensile displacements). As a consequence, the resistance of strands to the mechanical stimuli sharply diminishes.

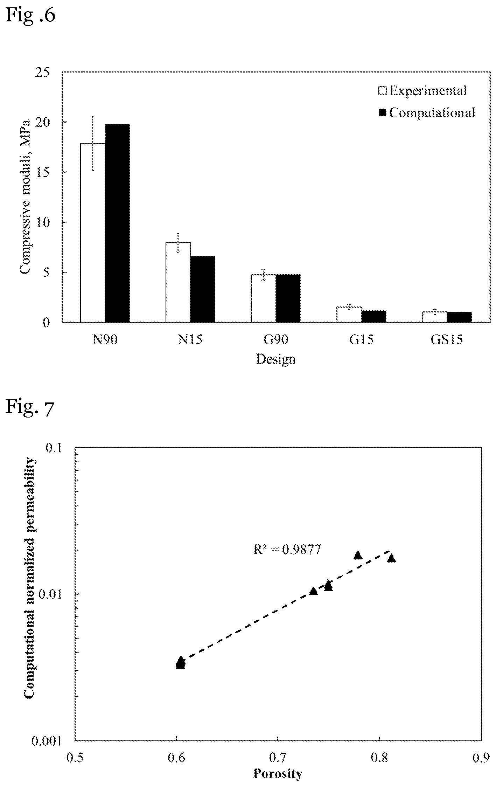

A comparison of experimental and computational compressive moduli for scaffolds (N90, N15, G90, G15, GS15) are shown in Fig. 6. It was found that compressive moduli from FEA predictions were in a good agreement with measurements from compression testing. The largest difference of 16.5% was observed for G15 scaffold. This can be acceptable due to the fact that the compressive modulus for G15 is as small as 1.17 MPa.

3.2 Transport behaviors

The study of mass transport behavior inside the scaffold was carried out in a virtual bioreactor model. The permeability was extracted from CFD simulations as shown in Fig. 7. It should be noted that the permeability was normalized by a reference permeability value ($9.13 \times 10^{-7}$ m$^2$) obtained from a scaffold with 100% porosity. A clear linear relation between the porosity and the normalized permeability was observed. To be specific, GS15 scaffold is found to have the highest permeability. In addition, the flow pattern was observed to be different for scaffolds with a similar level of permeability. The homogeneity degree of velocity field inside a scaffold was clearly higher for gradient and gradient + structures than that for a normal structure. In general, a better mixing induced from the flow results in a longer residence time for cells inside a scaffold. As a result, a cell has a high possibility to interact with other cells, struts of a scaffold, and attach eventually. In this case, the cell seeding efficiency would be improved. Furthermore, a more uniform distribution of cells through the scaffold may enhance cell proliferation and differentiation. Some evidence to this deduction can be found in previous study. It indicates that a scaffold with a gradient pore size can enhance the cell seeding efficiency and induce a more uniform distribution of cells. In brief, the flow pattern should be considered as a factor in the determination of the performance of different structural designs on cell transport behaviors inside a scaffold.

3.3 Characterization of 3d printed scaffolds

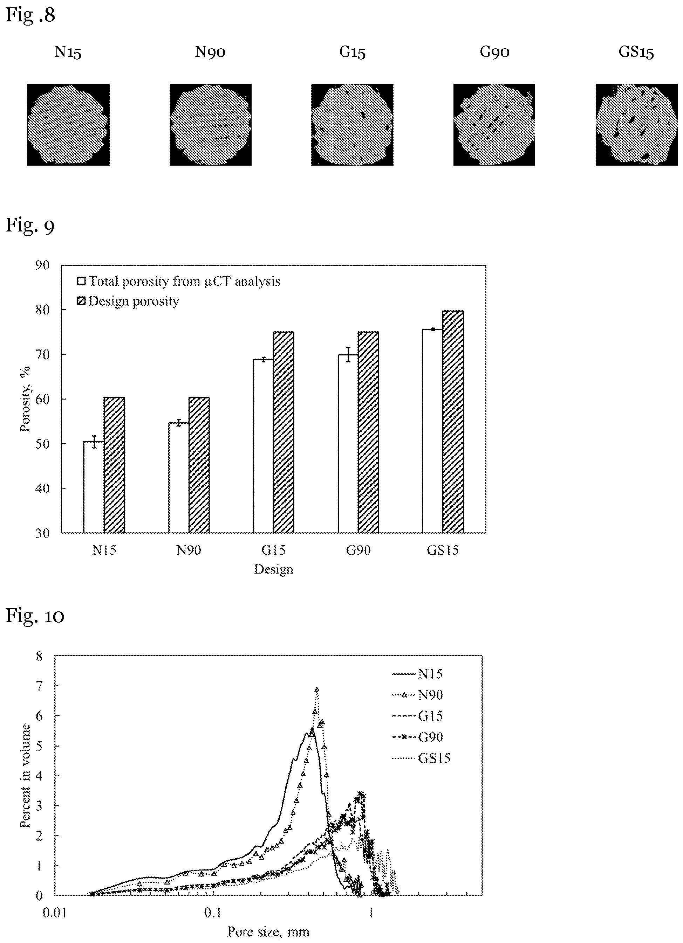

As shown in Fig. 8, five designed scaffolds were successfully produced by FDM 3D printer. The features of normal, gradient, gradient + staggered structure were captured in printed scaffolds. The mean strand thickness was detected to be around 0.4 mm for all scaffolds. This is in accordance with the used nozzle size of 0.4 mm. In addition, the total porosity of each scaffold was determined by μCT analysis. A comparison of the porosity between designed and printed scaffolds was made in Fig. 9. The porosity of printed scaffolds is in good agreement with that of designed scaffolds (N < G < GS). The N15 scaffolds have the lowest porosity of 50.4%, while the GS15 scaffolds have the highest porosity of 75.6%. Furthermore, the pore size distribution for printed scaffolds measured by μCT was presented in Fig. 10. For N15 and N90 scaffolds, a clear dominating size mode was observed. This indicates that the pore size is relatively homogeneously distributed within scaffolds with a normal structure. In contrast, for G15, G90 and GS15 scaffolds the pore size varies gradually and forms a weak dominating size mode. This observation suggests that a more heterogeneous pore size distribution is produced for scaffolds with a gradient/gradient+stagger structure. Despite that differences are detected from printed to designed scaffolds, FDM printing technology is a feasible fabrication method of polymer-based layer-by-layer type of scaffolds. It can be adapted for a broad range of thermoplastic materials where other rapid-prototyping methods, such as selective laser sintering (SLS), stereolithography (SLA), have limited material options. Additionally, it is a cost-effective method for the initial stage of scaffold design. In this sense, it is a good option to explore a compromise between material properties and transport behaviors in an efficient manner to produce prototypes for further studies.

### 4. Conclusions

In the present study, three types of structural designs (normal, gradient, gradient + stagger) were presented for soft tissue engineering with the assistance of computational simulation methods and FDM printing technology. Among these designs, a gradient combined with a stagger structure shows the best performance in terms of mechanical properties and mass transport. With this design, the scaffold possesses the lowest compressive/tensile modulus (approximately 0.23% of pure PCL) and the highest permeability. The computational compressive moduli for scaffolds were validated and in good agreement with measurements from compression testing. The largest difference was 16.5%. In addition, porosity effect is not the only key factor to influence the properties of a scaffold. As evidenced by results in this study, structure effect does play a role particularly in aspects of mechanical properties and mixing behaviors. Moreover, $\mu$CT analysis of printed scaffolds shows that FDM printing technology is capable of producing high-quality scaffolds from the designed structure. Lastly, the used method and findings in this study provides a priori knowledge of how architecture influence the performance of scaffolds.

### Example 2: Preparation of degradable poly($\varepsilon$-caprolactone-co-p-dioxanone) copolymers suitable for use in scaffolds for soft tissue engineering

### 1. Introduction

Poly($\varepsilon$-caprolactone), PCL, is a hydrophobic, semicrystalline resorbable polymer widely used for scaffolds manufacturing for tissue engineering applications. PCL is a prime candidate for melt-extrusion 3D printing of scaffolds. The low melting point, around 60 °C, is an advantage when the polymer is melt-processed, indeed, low processing temperature can be used avoiding chain degradation, which typically occurs when other degradable polymers are melt-processed. Moreover, PCL has higher thermal stability and a wider processing window but the high crystallinity, the hydrophobicity and the long degradation time (up to years) are serious limitations of PCL-based scaffolds. Poly(p-dioxanone) (PDX) is instead a semicrystalline poly(ether ester) that exhibits flexibility and pliability, as well as greater hydrophilicity and a faster degradation rate than PCL (6 months), but really poor thermal stability.

The aim of this study was to develop by bulk copolymerization of CL with p-dioxanone (DX) a copolymer suitable for the fabrication of scaffolds that could be processed in a manner similar to that for PCL, but have more rapid degradation rates than PCL while maintaining a certain degree of crystallinity, a low $T_m$ and high thermal stability.

### 2. Materials and methods

### 2.1. Bulk ring-opening polymerization

Copolymerization reaction of CL and DX were performed in bulk, using stannous octoate, $Sn(Oct)_2$, as catalyst and ethylene glycol as initiator. The ratio catalyst to initiator was 1:17 and the ratio monomers to initiator was calculated in order to have a $M_{n,th}$ = 80000 g mol$^{-1}$. The reaction temperature was 130 °C, while the copolymerization time was in the range 24-48 h.

### 2.2. Film preparation

Films of copolymers containing increasing amounts of DX were prepared with a thickness of ~200 $\mu$m by casting a polymer solution in $CHCl_3$ at a concentration of 100 g L$^{-1}$ in a 9 cm diameter Petri dish. Smaller size films were then cut into the desired shape for mechanical, contact angle and degradation tests.

### 2.3. Degradation tests

Degradation tests were performed over 120 days during which data point samples were collected at 4 times and each data point was analyzed in triplicate. Small films with a dimeter of 11 mm (30-40 mg weight) were dried in vacuum to a constant weight before tests were performed. The samples were immersed in vials containing 30 mL of sodium phosphate buffer, pH 7.4 and were maintained at 37 °C. The buffer solution was replaced every 3 weeks. Films were withdrawn from the incubation medium at scheduled times, washed carefully with distilled water, dried to a constant weight to calculate the mass loss (%) and then analyzed by SEC and NMR.

### 2.4. Copolymer and film characterization

NMR spectra of polymer and film samples were obtained in $CDCl_3$ at room temperature using a Bruker Avance

400 spectrometer ($^1$H: 400.13; $^{13}$C: 100.62 MHz). The resonances and coupling constants are reported in ppm ($\delta$) and Hz (J), respectively. $^1$H NMR spectra were referenced to the residual solvent proton at $\delta$ 7.26 ppm; $^{13}$C NMR spectra were referenced to the $^{13}$C signal of $CDCl_3$ at $\delta$ 77.16 ppm. Spectra were recorded using Bruker TopSpin v2.1 software. Data processing was performed using MestReNova v9.0.0 software. Molecular weights ($M_n$ and $M_w$) and dispersity (Đ) were measured by size exclusion chromatography (SEC). The measurements were performed at 35 $\underline{o}$C on a GPCMAX system equipped with an RI detector and three columns: one guard column (PLgel 5 $\mu$m Guard) and two linear columns (PLgel 5 $\mu$m Mixed-D), using $CHCl_3$ as the eluent (0.5 mL min-[1]). Narrow molecular weight polystyrene standards were used as standards and flow rate fluctuations were corrected using toluene as an internal standard.

Inherent viscosity (IV) measurements were performed on a Ubbelohde viscometer (capillary number 0, Capillary constant 0.009904) with the temperature set at 25 °C. For the analysis, 10 mg of polymer sample were dissolved in hexafluoroisopropanol (HFIP) and filtered using a 0.45 mm PTFE membrane. The results are expressed as the mean value of triplicate measurements.

Melting points ($T_m$) and enthalpies of fusion ($\Delta H_m$) of the polymeric samples were measured by differential scanning calorimetry (DSC) using aluminum pans and a Mettler Toledo DSC 1 Instruments apparatus that was calibrated with indium. Measurements were performed under nitrogen flow at a heating rate of 10 °C min-1 from -30 to 120 °C. DSC data are reported for the second heating cycle. The degree of crystallinity, $X_c$, was calculated from the DSC results by considering an enthalpy of fusion for an infinitely large PCL crystals of 136.1 J g-[1].

Thermogravimetric analysis (TGA) was performed in an $O_2$ atmosphere at a flowrate of 80 mL min-[1] in a temperature range from 25 °C to 500 °C at a heating rate of 10 °C min-[1] using a TGA/DSC 1 Instrument apparatus (Mettler Toledo, USA). The results are reported as the temperature at which 5% mass loss occurred ($T_{5\%}$).

The tensile mechanical properties of films (7 cm length, 0.45 cm width, 200 $\mu$m thickness) were measured at room temperature using an Instron 5944 tensile testing machine equipped with pneumatic grips. The free clamped length was 36 mm. A 500 N load cell was used and the cross-head speed was 500% mm min-[1] based on the initial length. All tensile properties are reported as the average of five measurements. Prior to analysis the specimens were conditioned for 24 h at 25 °C and 50% humidity.

Contact angle measurements were performed on films having a thickness of ~200 $\mu$m that were prepared by casting from a CHCl3 solution at a polymer concentration of 100 g L-[1] by placing a droplet of distilled water (MilliQ) on the surface and the angle was then estimated using CAM 2008 software. The reported values are the average of three measurements. Prior to analysis, the specimens were conditioned for 24 h at 25 °C and 50% humidity.

3. **Results and discussion**

Different polymerization runs were conducted by increasing the amount of DX in the feed from 0 to 20 mol%. $Sn(Oct)_2$ was used as the catalyst and ethylene glycol as the initiator and the reactions were performed in bulk at 130 °C (Table 2).

Table 2. Copolymerization of $\varepsilon$-caprolactone (CL) and p-dioxanone (DX).

| Entry | $f_{CL}$ (mol %)[a] | $f_{DX}$ (mol %)[a] | [monomers] : [Sn] : [i][b] | t (h) | Conversion (%)[c] | | Yield (%) | $F_{CL}$ (mol %)[d] | Fox (mol %)[d] | $M_n$ (kg mol-[1])[e] | Đ[e] | IV[f] (dL g-[1]) |
| --- | --- | --- | --- | --- | --- | --- | --- | --- | --- | --- | --- | --- |
| | | | | | CL | DX | | | | | | |
| **1** | 100 | - | 13500:1:17 | 24 | >99 | - | 99 | 100 | - | 176 | 1.7 | 3.37 |
| **2** | 90 | 10 | 13500:1:17 | 48 | 98 | 86 | 98 | 92 | 8 | 90.5 | 1.8 | 2.03 |
| **3** | 85 | 15 | 14000:1:17 | 48 | 99 | 96 | 99 | 87 | 13 | 57.0 | 1.9 | 1.70 |
| **4** | 80 | 20 | 15000:1:17 | 48 | 99 | 97 | 96 | 80 | 20 | 67.3 | 1.7 | 1.81 |

[a] Molar percentage of monomer in the feed.
[b] Ratio is calculated to produce a Mn,th ~ 80 kg mol-1.
[c] Determined from the 1H NMR spectra (CDCl3, 400 MHz) of the crude copolymerization mixture.
[d] Determined from the 1H NMR spectra (CDCl3, 400 MHz) of the purified copolymer.
[e] Determined by SEC (CHCl3, versus polystyrene standards).
[f] Determined by viscosimetry in HFIP at 25 °C.

The composition is close to the feed in all the cases and high conversion was achieved for both monomers. The experimental number average molecular weight values, $M_n$, determined by SEC, were close to the theoretical values. The inherent viscosity of the polymer samples was also determined and it tends to decrease with increasing DX content. The copolymer microstructure consisted of PCL chains containing randomly distributed, but isolated, DX units, by increasing the DX content, the length of the segments comprising consecutive CL units decreased.

All of the synthesised copolymers were semicrystalline materials. The $T_m$ decreased from 60.8 to 29.3 °C by increasing the DX content from 0 to 20 mol%, which is a consequence of the increased disorder along the polymer chains. A perfect linearly correlation was observed between the $T_m$ and the amount of CL in the copolymer. The degree of crystallinity varied in the range 35-19%, depending on the composition (Table 3).

**Table 3.** Thermal characterization.

| Entry | $F_{CL}$ (mol%)[a] | $F_{Dx}$ (mol%)[a] | $T_c$ (°C)[b] | $T_m$ (*C)[C] | $\Delta H$ (J g-1)[c] | $X_c$ (%)[d] | $T_{5\%}$ (°C)[e] |
|---|---|---|---|---|---|---|---|
| 1 | 100 | - | 27.9 | 60.8 | 47.6 | 35.0 | 278 |
| 2 | 92 | 8 | 20.7 | 48.8 | 40.7 | 29.9 | 266 |
| 3 | 87 | 13 | 16.4 | 43.5 | 40.2 | 29.5 | 275 |
| 4 | 80 | 20 | -9.8 | 29.23 | 26.3 | 19.3 | 256 |

[a] Determined from the $^1$H NMR spectra (CDCl$_3$, 400 MHz) of the purified copolymer.
[b] DSC data reported from the cooling run.
[c] DSC data reported from the second heating run.
[d] Calculated from DSC data assuming a $\Delta H°_{PCL}$ = 136.1 J g$^{-1}$.

The thermal stability of the synthesized copolymers was evaluated by TGA analysis. The temperature difference between the start of the degradation and the melting point was approximately 235-245 °C for all of the polymers, indicating that, similar to PCL, the copolymers can be melt processed in a wide range of temperatures. The copolymers retained a certain degree of crystallinity and showed good thermal stability comparable to that of PCL. Copolymer films showed stress-strain curves that are typical of soft and flexible thermoplastic materials. The mechanical properties are summarized in Table 4.

**Table 4.** Tensile properties of solvent cast copolymer.

| | Modulus (MPa) | | Strain at break (%) | | Stress at break (MPa) | |
|---|---|---|---|---|---|---|
| DX (mol%) | mean | ± | mean | ± | mean | ± |
| 0 | 365 | 30 | 930 | 100 | 30 | 5 |
| 8 | 290 | 15 | 730 | 100 | 11 | 2 |
| 13 | 320 | 35 | 450 | 180 | 12 | 1 |
| 20 | 82 | 15 | 1690 | 320 | 10 | 2 |

Contact angle measurements performed on solvent-cast films showed that by increasing the amount of DX in the copolymer, the hydrophobicity was reduced. PCL and the copolymer containing 8 mol% DX have contact angles greater than 90°, therefore films made from these polymers have a hydrophobic surface; increasing the amount of DX to 13 and 20 mol%, causes the contact angles to decrease to 86° and 80°, respectively, thus proving that DX moieties reduce the hydrophobicity of the polymer surface.

Hydrolytic degradation study performed in vitro showed that The $M_n$ values of the copolymer films decrease linearly with the degradation time; they are reduced by 50-80%, depending on the composition, after 120 days. Copolymer films containing 13 and 20 mol% DX have a $M_n$ that is lower than 20 kg mol-1 after 120 days. No variations in the Đ values were observed. The copolymer can reach a $M_n$ of 3000 g mol$^{-1}$ in 8-9 months. 3000 g mol$^{-1}$ is the limiting molecular weight, below which PCL oligomers are soluble and can diffuse out of the matrix, undergo intracellular resorption and lead to erosion of the implant.

## 4. **Conclusion**

Degradable polymers with low melting point suitable for the manufacturing of medical devises, mainly scaffolds for tissue engineering, that degrade in the human body, or other biological environments, and that can be melt processed at low temperature, thus, avoiding chain degradation during melt processing were developed. The polymers are random copolymers of ε-caprolactone (CL) and p-dioxanone (DX) having a content of DX from 5 to 20 mol%. Such materials combine the good processability and thermal stability of poly(caprolactone), PCL, with the hydrophilicity, good degradability and faster degradation of poly(p-dioxanone), PDX. Indeed, despite the low melting point, below 60 °C, the copolymers have high degradation temperature and thermal stability comparable to that of (PCL), and increased hydrophilicity as well as faster degradation time than PCL.

It is to be understood that the present disclosure is not restricted to the above-described exemplifying embodiments thereof and that several conceivable modifications of the present disclosure are possible within the scope of the following claims.

## Claims

1. A degradable scaffold (1) for use in tissue engineering, comprising:

   n layers (3), where n is an integer $\geq 4$;
   wherein each layer (3) extends in an x-y-plane;
   wherein each layer (3) has a height (H) in a z-direction perpendicular to the x-y-plane;
   wherein each layer (3) comprises m volume-building components (5) comprising degradable scaffold material and being aligned in r rows (7) in the x-y-plane, where m is an integer $\geq 2$, where r is an integer $\geq 2$;
   wherein each volume-building component (5) in any row (7) of a layer (3) is positioned at a distance (Dx) in the x-direction from any adjacent volume-building component (5) in any adjacent row (7) of the same layer (3);
   wherein the m volume-building components (5) of any group of layers (3) are distributed at an angle ($\alpha$) in the x-y-plane relative to the m volume-building components (5) of any adjacent group of layers (3);
   wherein the volume-building components (5) are configured to allow biological cells to attach thereto;
   **characterised in that**
   the degradable scaffold (1) comprises a gradient distribution of volume-building components (5) in the z-direction, wherein the distance (Dx) between any two adjacent groups of volume-building components (5) of any group of layers (3) is different from the distance (Dx) between any two adjacent groups of volume-building components (5) of any adjacent group of layers (3);
   and that
   the degradable scaffold (1) comprises a staggered distribution of volume-building components (5) of a layer (3) in the x-y-plane, wherein each volume-building component (5) in any row (7) of a layer (3) is positioned at a distance (Dysr) in the y-direction from any adjacent volume-building component (5) in the same row (7), and wherein each group of volume-building components (5) in any group of rows (7) of a layer (3) is positioned at a distance (Dyar) in the y-direction from any adjacent group of volume-building components (5) in any adjacent group of rows (7) of the same layer (3); wherein the height (H), the distance (Dx), the distance (Dysr), the distance (Dyar) and the angle ($\alpha$) together define a size and shape of pores in the scaffold (1), which pores are configured to allow biological cells to pass through the scaffold;
   wherein each group of layers comprises one layer or two or more adjacent layers, wherein each group of rows comprises one row or two or more adjacent rows, and wherein each group of volume-building components comprises one volume-building component or two or more adjacent volume-building components.

2. The degradable scaffold (1) according to claim 1, wherein the angle ($\alpha$) in the x-y-plane between any two adjacent groups of layers (3) is in a range of from >0° to 90°.

3. The degradable scaffold (1) according to any one of claims 1 or 2, wherein the gradient distribution of volume-building components (5) comprises:

   (a) the distance (Dx) between any two adjacent groups of volume-building components (5) of a group of layers (3) at a first end portion in the z-direction and/or at a second end portion in the z-direction of the scaffold (1) is larger than the distance (Dx) between any two adjacent groups of volume-building components (5) of a group of layers (3) at an intermediate portion in the z-direction of the scaffold (1); and/or
   (b) the distance (Dx) between any two adjacent groups of volume-building components (5) of a group of layers (3) at a first end portion in the z-direction of the scaffold (1) is larger than the distance (Dx) between any two adjacent groups of volume-building components (5) of a group of layers (3) at a second end portion in the z-direction of the scaffold (1).

4. The degradable scaffold (1) according to any one of claims 1-3, wherein the scaffold material of the volume-building components (5) comprises a polymer, a copolymer or a combination of different polymers and/or copolymers, preferably wherein the polymer(s) and/or copolymer(s) comprise one or more monomers selected from a group consisting of glycolide, lactide (D- or L-, meso or racemic mixture), trimethylene carbonate, $\varepsilon$-caprolactone, p-

dioxanone, β-butyrolactone and 1,5-dioxepan-2-one, or combinations thereof, more preferably wherein the scaffold material comprises a copolymer of ε-caprolactone and p-dioxanone.

5. The degradable scaffold (1) according to any one of claims 1-4, wherein the degradable scaffold material is a resorbable material.

6. A medical device for use in tissue engineering, comprising a degradable scaffold (1) according to any one of claims 1-5.

7. A method for preparing a degradable scaffold for tissue engineering,

the degradable scaffold comprising n layers extending in an x-y-plane, where n is an integer ≥ 4, each layer having a height (H) in a z-direction, and each layer comprising m volume-building components aligned in r rows in the x-y-plane, where m is an integer ≥ 2, where r is an integer ≥ 2;
the method comprising:

(A) providing a degradable scaffold material;
(B) preparing a degradable scaffold from the degradable scaffold material in an apparatus configured for additive manufacturing comprising a material source configured to deposit volume-building components of scaffold material, comprising:

(a) forming a first layer of the scaffold on top of a base plate of the apparatus configured for additive manufacturing, comprising:

i. depositing a first volume-building component of the scaffold material at a position on top of the base plate;
ii. moving the material source:

a. a distance (Dysr) in the y-direction, then a distance (Dx) in the x-direction and a distance (Dyar) in the y-direction, or
b. a distance (Dx) in the x-direction, or
c. a distance (Dx) in the x-direction and a distance (Dyar) in the y direction;

iii. depositing a subsequent volume-building component of the scaffold material at a subsequent position on the base plate, wherein the subsequent position is located:

a. at a distance (Dysr) in the y-direction from the position of the preceding volume-building component deposited, and wherein the subsequent volume-building component is aligned in the x-y-plane with the preceding volume-building component deposited, then at a distance (Dx) in the x-direction and a distance (Dyar) in the y-direction, and wherein the subsequent volume-building component is aligned in the x-y-plane with the preceding volume-building component deposited, or
b. at a distance (Dx) in the x-direction from the position of the preceding volume-building component deposited, and wherein the subsequent volume-building component is aligned in the x-y-plane with the preceding volume-building component deposited, or
c. at a distance (Dx) in the x-direction and at a distance (Dyar) in the y-direction from the position of the preceding volume-building component deposited, and wherein the subsequent volume-building component is aligned in the x-y-plane with the preceding volume-building component deposited;

iv. repeating steps ii-iii (m-2) times;

(b) forming (n-1) subsequent layers on top of the first layer of the scaffold in the apparatus configured for additive manufacturing, comprising:

i. moving the material source a distance (Dh) in the z-direction, equal to the height (H) of a layer;
ii. depositing a first volume-building component of the scaffold material at a position on top of the preceding layer, optionally at an angle (α) in the x-y-plane relative to the position of the first volume-building component of the preceding layer;

iii. moving the material source:

a. a distance (Dysr) in the y-direction then a distance (Dx) in the x-direction and a distance (Dyar) in the y-direction, or

b. a distance (Dx) in the x-direction, or

c. a distance (Dx) in the x-direction and a distance (Dyar) in the y-direction;

iv. depositing a subsequent volume-building component of the scaffold material at a subsequent position on top of the preceding layer, wherein the subsequent position is located:

a. at a distance (Dysr) in the y-direction, from the position of the preceding volume-building component deposited, and wherein the subsequent volume-building component is aligned in the x-y-plane with the preceding volume-building component deposited, a distance (Dx) in the x-direction, and a distance (Dyar) in the y-direction, and wherein the subsequent volume-building component is aligned in the x-y-plane with the preceding volume-building component deposited, or

b. at a distance (Dx) in the x-direction from the position of the preceding volume-building component deposited, and wherein the subsequent volume-building component is aligned in the x-y-plane with the preceding volume-building component deposited, and wherein the subsequent volume-building component is aligned in the x-y-plane with the preceding volume-building component deposited, or

c. at a distance (Dx) in the x-direction and at a distance (Dyar) in the y-direction from the position of the preceding volume-building component deposited, and wherein the subsequent volume-building component is aligned in the x-y-plane with the preceding volume-building component deposited;

v. repeating steps iii-iv (m-2) times;

vi. repeating steps i-v (n-2) times;

provided that the distance (Dx) in any group of layers is different from the distance (Dx) of the preceding group of layers;

provided that the m volume-building components of any group of layers are distributed at an angle ($\alpha$) in the x-y-plane relative to the m volume-building components of any adjacent group of layers;

thereby preparing a degradable scaffold comprising a gradient distribution of volume-building components in the z-direction of the scaffold, and a staggered distribution of volume-building components in the x-y-plane of the scaffold;

wherein the height (H), the distance (Dx), the distance (Dysr), the distance (Dyar) and the angle ($\alpha$) together define a size and shape of pores in the scaffold.

8. The method according to claim 7, wherein the degradable scaffold material comprises a polymer, a copolymer or a combination of different polymers and/or copolymers, preferably wherein the polymer(s) and/or copolymer(s) comprise one or more monomers selected from a group consisting of glycolide, lactide (D- or L-, meso or racemic mixture), trimethylene carbonate, $\varepsilon$-caprolactone, p-dioxanone, $\beta$-butyrolactone and 1,5-dioxepan-2-one, or combinations thereof, more preferably wherein the scaffold material comprises a copolymer of $\varepsilon$-caprolactone and p-dioxanone.

9. The method according to claim 7 or 8, wherein the degradable scaffold material is a resorbable material and the step of providing a degradable scaffold material comprises providing said resorbable material.

10. Use of a degradable scaffold according to any one of claims 1-5 in an *in vitro* cell culture system, in an *in vitro* method for culturing of cells and/or in an *in vitro* method for regenerating tissue.

**Patentansprüche**

1. Abbaubares Gerüst (1) zur Verwendung in der Gewebezüchtung, umfassend:

   n Schichten (3), wobei n eine ganze Zahl $\geq 4$ ist;
   wobei sich jede Schicht (3) in einer x-y-Ebene erstreckt;
   wobei jede Schicht (3) eine Höhe (H) in einer z-Richtung senkrecht zur x-y-Ebene aufweist;
   wobei jede Schicht (3) m volumenbildende Komponenten (5) umfasst, die aus abbaubarem Gerüstmaterial bestehen und in r Reihen (7) in der x-y-Ebene angeordnet sind, wobei m eine ganze Zahl $\geq 2$ ist, wobei r eine ganze Zahl $\geq 2$ ist;
   wobei jede volumenbildende Komponente (5) in einer beliebigen Reihe (7) einer Schicht (3) in einer Distanz (Dx) in der x-Richtung von jeder benachbarten volumenbildenden Komponente (5) in einer beliebigen benachbarten Reihe (7) der gleichen Schicht (3) positioniert ist;
   wobei die m volumenbildenden Komponenten (5) einer beliebigen Gruppe von Schichten (3) in einem Winkel ($\alpha$) in der x-y-Ebene relativ zu den m volumenbildenden Komponenten (5) einer beliebigen benachbarten Gruppe von Schichten (3) verteilt sind;
   wobei die volumenbildenden Komponenten (5) so ausgebildet sind, dass sich biologische Zellen daran anheften können;
   **dadurch gekennzeichnet, dass**
   das abbaubare Gerüst (1) eine Gradientenverteilung von volumenbildenden Komponenten (5) in der z-Richtung umfasst, wobei die Distanz (Dx) zwischen zwei beliebigen benachbarten Gruppen von volumenbildenden Komponenten (5) einer beliebigen Gruppe von Schichten (3) sich von der Distanz (Dx) zwischen zwei beliebigen benachbarten Gruppen von volumenbildenden Komponenten (5) einer beliebigen benachbarten Gruppe von Schichten (3) unterscheidet;
   und dadurch, dass
   das abbaubare Gerüst (1) eine versetzte Verteilung von volumenbildenden Komponenten (5) einer Schicht (3) in der x-y-Ebene umfasst, wobei jede volumenbildende Komponente (5) in jeder Reihe (7) einer Schicht (3) in einer Distanz (Dysr) in der y-Richtung von jeder benachbarten volumenbildenden Komponente (5) in der gleichen Reihe (7) positioniert ist, und wobei jede Gruppe von volumenbildenden Komponenten (5) in jeder Gruppe von Reihen (7) einer Schicht (3) in einer Distanz (Dyar) in der y-Richtung von jeder benachbarten Gruppe von volumenbildenden Komponenten (5) in jeder benachbarten Gruppe von Reihen (7) der gleichen Schicht (3) positioniert ist; die Gruppe mehr als eine volumenbildende Komponente umfasst, wobei die volumenbildenden Komponenten der Gruppe aneinander angrenzen; wobei die Höhe (H), die Distanz (Dx), die Distanz (Dysr), die Distanz (Dyar) und der Winkel ($\alpha$) zusammen eine Größe und Form von Poren im Gerüst (1) definieren, wobei die Poren so beschaffen sind, dass biologische Zellen durch das Gerüst hindurch gelangen können.

2. Abbaubares Gerüst (1) nach Anspruch 1, wobei der Winkel ($\alpha$) in der x-y-Ebene zwischen zwei beliebigen benachbarten Gruppen von Schichten (3) in einem Bereich von $\geq 0°$ bis 90° liegt.

3. Abbaubares Gerüst (1) nach einem der Ansprüche 1 oder 2, wobei die Gradientenverteilung der volumenbildenden Komponenten (5) umfasst:

   (a) die Distanz (Dx) zwischen zwei beliebigen benachbarten Gruppen von volumenbildenden Komponenten (5) einer Gruppe von Schichten (3) an einem ersten Endabschnitt in z-Richtung und/oder an einem zweiten Endabschnitt in z-Richtung des Gerüsts (1) ist größer als die Distanz (Dx) zwischen zwei beliebigen benachbarten Gruppen von volumenbildenden Komponenten (5) einer Gruppe von Schichten (3) an einem Zwischenabschnitt in z-Richtung des Gerüsts (1); und/oder
   (b) die Distanz (Dx) zwischen zwei beliebigen benachbarten Gruppen von volumenbildenden Komponenten (5) einer Gruppe von Schichten (3) an einem ersten Endabschnitt in der z-Richtung des Gerüsts (1) ist größer als die Distanz (Dx) zwischen zwei beliebigen benachbarten Gruppen von volumenbildenden Komponenten (5) einer Gruppe von Schichten (3) an einem zweiten Endabschnitt in der z-Richtung des Gerüsts (1).

4. Abbaubares Gerüst (1) nach einem der Ansprüche 1 bis 3, wobei das Gerüstmaterial der volumenbildenden Komponenten (5) ein Polymer, ein Copolymer oder eine Kombination verschiedener Polymere und/oder Copolymere umfasst, wobei das/die Polymer(e) und/oder Copolymer(e) vorzugsweise ein oder mehrere Monomere umfassen, ausgewählt aus einer Gruppe bestehend aus Glycolid, Lactid (D- oder **L-,** Meso- oder Racemat-Gemisch), Trimethylencarbonat, $\varepsilon$-Caprolacton, *p*-Dioxanon, $\beta$-Butyrolacton und 1,5-Dioxepan-2-on oder Kombinationen davon, wobei das Gerüstmaterial vorzugsweise ein Copolymer aus $\varepsilon$-Caprolacton und *p*-Dioxanon.

**5.** Abbaubares Gerüst (1) nach einem der Ansprüche 1-4, wobei das abbaubare Gerüstmaterial ein resorbierbares Material ist.

**6.** Medizinische Vorrichtung zur Verwendung in der Gewebezüchtung, umfassend ein abbaubares Gerüst (1) nach einem der Ansprüche 1 bis 5.

**7.** Verfahren zum Herstellen eines abbaubaren Gerüsts für die Gewebezüchtung,

wobei das abbaubare Gerüst n Schichten umfasst, die sich in einer x-y-Ebene erstrecken, wobei n eine ganze Zahl $\geq$ 4 ist, wobei jede Schicht eine Höhe (H) in einer z-Richtung aufweist, und wobei jede Schicht m volumenbildende Komponenten umfasst, die in r Reihen in der x-y-Ebene ausgerichtet sind, wobei m eine ganze Zahl $\geq$ 2 ist, wobei r eine ganze Zahl $\geq$ 2 ist;
das Verfahren umfassend:

(A) Bereitstellen eines abbaubaren Gerüstmaterials;
(B) Herstellen eines abbaubaren Gerüsts aus dem abbaubaren Gerüstmaterial in einer Vorrichtung, die für die additive Fertigung konfiguriert ist und eine Materialquelle umfasst, die zum Ablagern von volumenbildenden Komponenten aus Gerüstmaterial konfiguriert ist, umfassend:

(a) Bilden einer ersten Schicht des Gerüsts auf der Oberseite einer Grundplatte der für die additive Fertigung konfigurierten Vorrichtung, umfassend:

i. Ablagern einer ersten volumenbildenden Komponente des Gerüstmaterials an einer Position auf der Oberseite der Grundplatte;
ii. Bewegen der Materialquelle:

a. eine Distanz (Dysr) in y-Richtung, dann eine Distanz (Dx) in x-Richtung und eine Distanz (Dyar) in y-Richtung, oder
b. eine Distanz (Dx) in x-Richtung, oder
c. eine Distanz (Dx) in der x-Richtung und eine Distanz (Dyar) in der y-Richtung;

iii. Ablagern einer nachfolgenden volumenbildenden Komponente des Gerüstmaterials an einer nachfolgenden Position auf der Grundplatte, wobei sich die nachfolgende Position befindet:

a. in einer Distanz (Dysr) in y-Richtung von der Position der vorhergehenden abgelagerten volumenbildenden Komponente, und wobei die nachfolgende volumenbildende Komponente in der x-y-Ebene mit der vorhergehenden abgelagerten volumenbildenden Komponente ausgerichtet ist, dann in einer Distanz (Dx) in x-Richtung und einer Distanz (Dyar) in y-Richtung, und wobei die nachfolgende volumenbildende Komponente in der x-y-Ebene mit der vorhergehenden abgelagerten volumenbildenden Komponente ausgerichtet ist, oder
b. in einer Distanz (Dx) in x-Richtung von der Position der vorhergehenden abgelagerten volumenbildenden Komponente, und wobei die nachfolgende volumenbildende Komponente in der x-y-Ebene mit der vorhergehenden abgelagerten volumenbildenden Komponente ausgerichtet ist, oder
c. in einer Distanz (Dx) in x-Richtung und in einer Distanz (Dyar) in y-Richtung von der Position der vorhergehenden abgelagerten volumenbildenden Komponente, und wobei die nachfolgende volumenbildende Komponente in der x-y-Ebene mit der vorhergehenden abgelagerten volumenbildenden Komponente ausgerichtet ist;

iv. Wiederholen der Schritte ii-iii (m-2) Mal.

(b) Bilden von (n-1) nachfolgenden Schichten auf der Oberseite der ersten Schicht des Gerüsts in der für die additive Fertigung konfigurierten Vorrichtung, umfassend:

i. Bewegen der Materialquelle um eine Distanz (Dh) in der z-Richtung, die der Höhe (H) einer Schicht entspricht;
ii. Ablagern einer ersten volumenbildenden Komponente des Gerüstmaterials an einer Position auf der Oberseite der vorhergehenden Schicht, optional in einem Winkel ($\alpha$) in der x-y-Ebene relativ zur Position der ersten volumenbildenden Komponente der vorhergehenden Schicht;
iii. Bewegen der Materialquelle:

a. eine Distanz (Dysr) in y-Richtung, dann eine Distanz (Dx) in x-Richtung und eine Distanz (Dyar) in y-Richtung, oder
b. eine Distanz (Dx) in x-Richtung, oder
c. eine Distanz (Dx) in der x-Richtung und eine Distanz (Dyar) in der y-Richtung;

iv. Ablagern einer nachfolgenden volumenbildenden Komponente des Gerüstmaterials an einer nachfolgenden Position auf der vorhergehenden Schicht, wobei sich die nachfolgende Position befindet:

a. in einer Distanz (Dysr) in y-Richtung von der Position der vorhergehenden abgelagerten volumenbildenden Komponente, und wobei die nachfolgende volumenbildende Komponente in der x-y-Ebene mit der vorhergehenden abgelagerten volumenbildenden Komponente ausgerichtet ist, einer Distanz (Dx) in x-Richtung und einer Distanz (Dyar) in y-Richtung, und wobei die nachfolgende volumenbildende Komponente in der x-y-Ebene mit der vorhergehenden abgelagerten volumenbildenden Komponente ausgerichtet ist, oder
b. in einer Distanz (Dx) in der x-Richtung von der Position der vorhergehenden abgelagerten volumenbildenden Komponente, und wobei die nachfolgende volumenbildende Komponente in der x-y-Ebene mit der vorhergehenden abgelagerten volumenbildenden Komponente ausgerichtet ist, und wobei die nachfolgende volumenbildende Komponente in der x-y-Ebene mit der vorhergehenden abgelagerten volumenbildenden Komponente ausgerichtet ist, oder
c. in einer Distanz (Dx) in x-Richtung und in einer Distanz (Dyar) in y-Richtung von der Position der vorhergehenden abgelagerten volumenbildenden Komponente, und wobei die nachfolgende volumenbildende Komponente in der x-y-Ebene mit der vorhergehenden abgelagerten volumenbildenden Komponente ausgerichtet ist;

v. Wiederholen der Schritte iii-iv (m-2) Mal;
vi. Wiederholen der Schritte i-v (n-2) Mal;

vorausgesetzt, dass die Distanz (Dx) in einer beliebigen Schichtgruppe sich von der Distanz (Dx) der vorhergehenden Schichtgruppe unterscheidet;
vorausgesetzt, dass die m volumenbildenden Komponenten einer beliebigen Schichtgruppe in einem Winkel ($\alpha$) in der x-y-Ebene relativ zu den m volumenbildenden Komponenten einer beliebigen benachbarten Schichtgruppe verteilt sind;
dadurch Herstellen eines abbaubaren Gerüsts, umfassend eine Gradientenverteilung von volumenbildenden Komponenten in der z-Richtung des Gerüsts und eine versetzte Verteilung von volumenbildenden Komponenten in der x-y-Ebene des Gerüsts;
wobei die Höhe (H), die Distanz (Dx), die Distanz (Dysr), die Distanz (Dyar) und der Winkel ($\alpha$) zusammen eine Größe und Form von Poren im Gerüst definieren.

8. Verfahren nach Anspruch 7, wobei das abbaubare Gerüstmaterial ein Polymer, ein Copolymer oder eine Kombination verschiedener Polymere und/oder Copolymere umfasst, wobei das/die Polymer(e) und/oder Copolymer(e) vorzugsweise ein oder mehrere Monomere umfassen, ausgewählt aus der Gruppe bestehend aus Glycolid, Lactid (D- oder **L-**, Meso- oder Racemat-Gemisch), Trimethylencarbonat, $\epsilon$-Caprolacton, $p$-Dioxanon, $\beta$-Butyrolacton und 1,5-Dioxepan-2-on oder Kombinationen davon, wobei das Gerüstmaterial vorzugsweise ein Copolymer aus $\epsilon$-Caprolacton und $p$-Dioxanon.

9. Verfahren nach Anspruch 7 oder 8, wobei das abbaubare Gerüstmaterial ein resorbierbares Material ist und der Schritt der Bereitstellung eines abbaubaren Gerüstmaterials die Bereitstellung des resorbierbaren Materials umfasst.

10. Verwendung eines abbaubaren Gerüsts nach einem der Ansprüche 1 bis 5 in einem *in* vitro-Zellkultursystem, in einem *in* vitro-Verfahren zur Kultivierung von Zellen und/oder in einem *in* vitro-Verfahren zur Geweberegeneration.

**Revendications**

1. Échafaudage dégradable (1) destiné à être utilisé en ingénierie tissulaire, comprenant :

   n couches (3), où n est un nombre entier $\geq 4$ ;
   dans lequel chaque couche (3) s'étend dans un plan x-y ;
   dans lequel chaque couche (3) a une hauteur (H) dans une direction z perpendiculaire au plan x-y ;
   dans lequel chaque couche (3) comprend m composants de construction en volume (5) comprenant un matériau d'échafaudage dégradable et alignés en r rangées (7) dans le plan x-y, où m est un nombre entier $\geq 2$, où r est un nombre entier $\geq 2$ ;
   dans lequel chaque composant de construction en volume (5) d'une rangée (7) quelconque d'une couche (3) est positionné à une distance (Dx) dans la direction x d'un composant de construction en volume (5) adjacent quelconque d'une rangée (7) adjacente quelconque de la même couche (3) ;
   dans lequel les m composants de construction en volume (5) d'un groupe de couches (3) quelconque sont répartis selon un angle ($\alpha$) dans le plan x-y par rapport aux m composants de construction en volume (5) d'un groupe de couches (3) adjacent quelconque ;
   dans lequel les composants de construction en volume (5) sont conçus pour permettre à des cellules biologiques de s'y attacher ;
   **caractérisé en ce que**
   l'échafaudage dégradable (1) comprend une distribution en gradient de composants de construction en volume (5) dans la direction z, dans lequel la distance (Dx) entre deux groupes adjacents quelconques de composants de construction en volume (5) d'un groupe de couches (3) quelconque est différente de la distance (Dx) entre deux groupes adjacents quelconques de composants de construction en volume (5) d'un groupe de couches (3) adjacent quelconque ;
   **et en ce que**
   l'échafaudage dégradable (1) comprend une distribution échelonnée de composants de construction en volume (5) d'une couche (3) dans le plan x-y, dans lequel chaque composant de construction en volume (5) dans une rangée (7) quelconque d'une couche (3) est positionné à une distance (Dysr) dans la direction y d'un composant de construction en volume (5) adjacent quelconque dans la même rangée (7), et dans lequel chaque groupe de composants de construction en volume (5) dans un groupe de rangées (7) quelconque d'une couche (3) est positionné à une distance (Dyar) dans la direction y d'un groupe de composants de construction en volume (5) adjacent quelconque dans un groupe de rangées (7) adjacent quelconque de la même couche (3) ; le groupe comprend plus d'un composant de construction en volume, dans lequel les composants de construction en volume du groupe sont adjacents les uns aux autres ; dans lequel la hauteur (H), la distance (Dx), la distance (Dysr), la distance (Dyar) et l'angle ($\alpha$) définissent ensemble une taille et une forme de pores dans l'échafaudage (1), lesquels pores sont conçus pour permettre à des cellules biologiques de passer à travers l'échafaudage.

2. Échafaudage dégradable (1) selon la revendication 1, dans lequel l'angle ($\alpha$) dans le plan x-y entre deux groupes de couches (3) adjacents quelconques est compris dans une plage allant de > 0° à 90°.

3. Échafaudage dégradable (1) selon l'une quelconque des revendications 1 ou 2, dans lequel la distribution en gradient de composants de construction en volume (5) comprend :

   (a) la distance (Dx) entre deux groupes adjacents quelconques de composants de construction en volume (5) d'un groupe de couches (3) au niveau d'une première partie d'extrémité dans la direction z et/ou au niveau d'une seconde partie d'extrémité dans la direction z de l'échafaudage (1) est plus grande que la distance (Dx) entre deux groupes adjacents quelconques de composants de construction en volume (5) d'un groupe de couches (3) au niveau d'une partie intermédiaire dans la direction z de l'échafaudage (1) ; et/ou
   (b) la distance (Dx) entre deux groupes adjacents quelconques de composants de construction en volume (5) d'un groupe de couches (3) au niveau d'une première partie d'extrémité dans la direction z de l'échafaudage (1) est plus grande que la distance (Dx) entre deux groupes adjacents quelconques de composants de construction en volume (5) d'un groupe de couches (3) au niveau d'une seconde partie d'extrémité dans la direction z de l'échafaudage (1).

4. Échafaudage dégradable (1) selon l'une quelconque des revendications 1 à 3, dans lequel le matériau d'échafaudage des composants de construction en volume (5) comprend un polymère, un copolymère ou une combinaison de différents polymères et/ou copolymères, de préférence dans lequel le(s) polymère(s) et/ou copolymère(s) comprennent un ou plusieurs monomères choisis dans un groupe constitué de glycolide, lactide (D- ou L-, méso ou mélange racémique), carbonate de triméthylène, ε-caprolactone, p-dioxanone, β-butyrolactone et 1,5-dioxépan-2-one, ou combinaisons de ceux-ci, plus préférablement dans lequel le matériau d'échafaudage comprend un copolymère d'ε-caprolactone et de p-dioxanone.

**5.** Échafaudage dégradable (1) selon l'une quelconque des revendications 1 à 4, dans lequel le matériau d'échafaudage dégradable est un matériau résorbable.

**6.** Dispositif médical destiné à être utilisé en ingénierie tissulaire, comprenant un échafaudage dégradable (1) selon l'une quelconque des revendications 1 à 5.

**7.** Procédé de préparation d'un échafaudage dégradable d'ingénierie tissulaire,

l'échafaudage dégradable comprenant n couches s'étendant dans un plan x-y, où n est un nombre entier ≥ 4, chaque couche ayant une hauteur (H) dans une direction z, et chaque couche comprenant m composants de construction en volume alignés en r rangées dans le plan x-y, où m est un nombre entier ≥ 2, où r est un nombre entier ≥ 2 ;
le procédé comprenant :

(A) la fourniture d'un matériau d'échafaudage dégradable ;
(B) la préparation d'un échafaudage dégradable à partir du matériau d'échafaudage dégradable dans un appareil configuré pour la fabrication additive comprenant une source de matériau configurée pour déposer des composants de construction en volume de matériau d'échafaudage, comprenant :

(a) la formation d'une première couche de l'échafaudage sur une plaque de base de l'appareil configuré pour une fabrication additive, comprenant :

i. le dépôt d'un premier composant de construction en volume du matériau d'échafaudage au niveau d'une position sur la plaque de base ;
ii. le déplacement de la source de matériau :

a. d'une distance (Dysr) dans la direction y, puis d'une distance (Dx) dans la direction x et d'une distance (Dyar) dans la direction y, ou
b. d'une distance (Dx) dans la direction x, ou
c. d'une distance (Dx) dans la direction x et d'une distance (Dyar) dans la direction y ;

iii. le dépôt d'un composant de construction en volume ultérieur du matériau d'échafaudage au niveau d'une position ultérieure sur la plaque de base, dans lequel la position ultérieure est située :

a. à une distance (Dysr) dans la direction y de la position du composant de construction en volume précédent déposé, et dans lequel le composant de construction en volume ultérieur est aligné dans le plan x-y avec le composant de construction en volume précédent déposé, puis à une distance (Dx) dans la direction x et à une distance (Dyar) dans la direction y, et dans lequel le composant de construction en volume ultérieur est aligné dans le plan x-y avec le composant de construction en volume précédent déposé, ou
b. à une distance (Dx) dans la direction x de la position du composant de construction en volume précédent déposé, et dans lequel le composant de construction en volume ultérieur est aligné dans le plan x-y avec le composant de construction en volume précédent déposé, ou
c. à une distance (Dx) dans la direction x et à une distance (Dyar) dans la direction y de la position du composant de construction en volume précédent déposé, et dans lequel le composant de construction en volume ultérieur est aligné dans le plan x-y avec le composant de construction en volume précédent déposé ;

iv. la répétition des étapes ii-iii (m-2) fois ;

(b) la formation de (n-1) couches ultérieures sur la première couche de l'échafaudage dans l'appareil configuré pour une fabrication additive, comprenant :

i. le déplacement de la source de matériau d'une distance (Dh) dans la direction z, égale à la hauteur (H) d'une couche ;

ii. le dépôt d'un premier composant de construction en volume du matériau d'échafaudage au niveau d'une position sur la couche précédente, facultativement à un angle ($\alpha$) dans le plan x-y par rapport à la position du premier composant de construction en volume de la couche précédente ;

iii. le déplacement de la source de matériau :

    a. d'une distance (Dysr) dans la direction y, puis d'une distance (Dx) dans la direction x et d'une distance (Dyar) dans la direction y, ou
    b. d'une distance (Dx) dans la direction x, ou
    c. d'une distance (Dx) dans la direction x et d'une distance (Dyar) dans la direction y ;

iv. le dépôt d'un composant de construction en volume ultérieur du matériau d'échafaudage au niveau d'une position ultérieure sur la couche précédente, dans lequel la position ultérieure est située :

    a. à une distance (Dysr) dans la direction y de la position du composant de construction en volume précédent déposé, et dans lequel le composant de construction en volume ultérieur est aligné dans le plan x-y avec le composant de construction en volume précédent déposé, à une distance (Dx) dans la direction x, et à une distance (Dyar) dans la direction y, et dans lequel le composant de construction en volume ultérieur est aligné dans le plan x-y avec le composant de construction en volume précédent déposé, ou
    b. à une distance (Dx) dans la direction x de la position du composant de construction en volume précédent déposé, et dans lequel le composant de construction en volume ultérieur est aligné dans le plan x-y avec le composant de construction en volume précédent déposé, et dans lequel le composant de construction en volume ultérieur est aligné dans le plan x-y avec le composant de construction en volume précédent déposé, ou
    c. à une distance (Dx) dans la direction x et à une distance (Dyar) dans la direction y de la position du composant de construction en volume précédent déposé, et dans lequel le composant de construction en volume ultérieur est aligné dans le plan x-y avec le composant de construction en volume précédent déposé ;

v. la répétition des étapes iii-iv (m-2) fois ;
vi. la répétition des étapes i-v (n-2) fois ;

à condition que la distance (Dx) d'un groupe de couches quelconque soit différente de la distance (Dx) du groupe de couches précédent ;

à condition que les m composants de construction en volume d'un groupe de couches quelconque soient répartis selon un angle ($\alpha$) dans le plan x-y par rapport aux m composants de construction en volume d'un groupe de couches adjacent quelconque ;

permettant ainsi de préparer un échafaudage dégradable comprenant une distribution en gradient de composants de construction en volume dans la direction z de l'échafaudage, et une distribution échelonnée de composants de construction en volume dans le plan x-y de l'échafaudage ;

dans lequel la hauteur (H), la distance (Dx), la distance (Dysr), la distance (Dyar) et l'angle ($\alpha$) définissent ensemble une taille et une forme de pores dans l'échafaudage.

**8.** Procédé selon la revendication 7, dans lequel le matériau d'échafaudage dégradable comprend un polymère, un copolymère ou une combinaison de différents polymères et/ou copolymères, de préférence dans lequel le(s) polymère(s) et/ou copolymère(s) comprennent un ou plusieurs monomères choisis dans un groupe constitué de glycolide, lactide (D- ou L-, méso ou mélange racémique), carbonate de triméthylène, $\varepsilon$-caprolactone, p-dioxanone, $\beta$-butyrolactone et 1,5-dioxépan-2-one, ou combinaisons de ceux-ci, plus préférablement dans lequel le matériau d'échafaudage comprend un copolymère d'$\varepsilon$-caprolactone et de p-dioxanone.

**9.** Procédé selon la revendication 7 ou 8, dans lequel le matériau d'échafaudage dégradable est un matériau résorbable et l'étape de fourniture d'un matériau d'échafaudage dégradable comprend la fourniture dudit matériau résorbable.

**10.** Utilisation d'un échafaudage dégradable selon l'une quelconque des revendications 1 à 5 dans un système de culture cellulaire *in vitro,* dans un procédé de culture cellulaire *in vitro* et/ou dans un procédé de régénération tissulaire *in vitro.*

Fig.1

Fig. 2

(a)

(k)

(b)

(l)

(c)

(m)

(d)

(n)

(e)

(o)

Fig. 2

(f)　　　　　　　　　　　　(p)

(g)　　　　　　　　　　　　(q)

(h)　　　　　　　　　　　　(r)

(i)　　　　　　　　　　　　(s)

(j)　　　　　　　　　　　　(t)

Fig. 3

EP 3 946 147 B1

31

A — Providing scaffold material

B(a)i — Depositing first volume-building component (VBC) of first layer

x(m-2)

B(a)ii(a) — Moving Dysr

B(a)ii(b) — Moving Dx

B(a)ii(c) — Moving Dx and Dyar

B(a)iii(a) — Depositing VBC

B(a)iii(b) — Depositing VBC

B(a)iii(c) — Depositing VBC

x(n-2)

B(b)i — Moving Dh

B(b)ii — Depositing first VBC of subsequent layer (optionally α)

x(m-2)

B(b)iii(a) — Moving Dysr

B(b)iii(b) — Moving Dx

B(b)iii(c) — Moving Dx and Dyar

B(b)iv(a) — Depositing VBC

B(b)iv(b) — Depositing VBC

B(b)iv(c) — Depositing VBC

Fig. 4

Fig. 5

Fig .6

Fig. 7

Fig .8

N15          N90          G15          G90          GS15

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016374431 A1
- WO 2014095872 A1
- US 2021186678 A1

**Non-patent literature cited in the description**

- **PEREZ et al.** *Materials Science and Engineering C,* 2016, vol. 61, 922-939
- **SLESARENKO et al.** *Smart materials and structures,* 2017, vol. 26 (3), 35053